(19) Europäisches Patentamt
European Patent Office
Office européen des brevets

(11) EP 2 157 789 A1

(12) **EUROPEAN PATENT APPLICATION**
published in accordance with Art. 153(4) EPC

(43) Date of publication:
**24.02.2010 Bulletin 2010/08**

(21) Application number: **08753041.6**

(22) Date of filing: **21.05.2008**

(51) Int Cl.:
***H04N 5/76*** *(2006.01)* ***A61B 1/00*** *(2006.01)*
***A61B 1/04*** *(2006.01)* ***A61B 1/06*** *(2006.01)*

(86) International application number:
**PCT/JP2008/059364**

(87) International publication number:
**WO 2008/155974 (24.12.2008 Gazette 2008/52)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **20.06.2007 JP 2007163033**

(71) Applicant: **Olympus Corporation**
**Tokyo 151-0072 (JP)**

(72) Inventor: **MATSUZAKI, Hiroshi**
**Tokyo 151-0072 (JP)**

(74) Representative: **Schmidt, Steffen**
**Wuesthoff & Wuesthoff**
**Patentanwälte**
**Schweigerstrasse 2**
**81541 München (DE)**

## (54) IMAGE EXTRACTING APPARATUS, IMAGE EXTRACTING PROGRAM, AND IMAGE EXTRACTING METHOD

(57) The image extracting apparatus includes the scene-change-image extracting unit (12) that extracts a scene change image located at a position at which a scene is changed from an image sequence acquired in time series by using a predetermined extraction condition, a comparing unit (132) that compares the scene change image with an adjacent image that is acquired at a time adjacent to the time at which the scene change image is acquired within a predetermined time-series range, and a summary image extracting unit (13) that extracts one of the scene change image and the adjacent image corresponding to the scene change image as a summary image by using the result compared by the comparing unit (132).

FIG.1

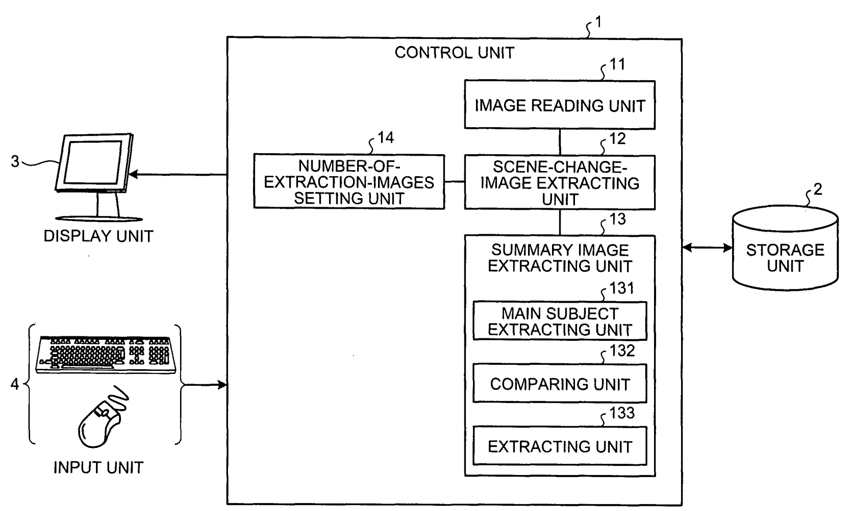


EP 2 157 789 A1

## Description

TECHNICAL FIELD

The present invention relates to an image extracting apparatus that extracts a scene change position or a valid image within a sequence of images that are continuously captured or a sequence of moving-image frames, an image extracting program therefor, and an image extracting method therefor.

BACKGROUND ART

Conventional image extracting apparatuses that extract an image (hereinafter, "scene change image") located at a position at which a scene is changed within a sequence of consecutive images such as a moving image are well known (see Patent Document 1). Even if a user does not view all images included in the sequence of images, the user can grasp the entire contents of the sequence of images in a short time and can easily find a desired scene or image by viewing a predetermined number of scene change images extracted by the image extracting apparatus.
Furthermore, an image extracting apparatus that extracts a plurality of images located between scene change frames in addition to the scene change frames by using a predetermined extraction condition is well known (see Patent Documents 2 and 3).
[Patent Document 1] Japanese Patent Application Laid-open No. 2001-54055
[Patent Document 2] Japanese Patent Application Laid-open No. 2006-217045
[Patent Document 3] Japanese Patent Application Laid-open No. 2006-217046

DISCLOSURE OF INVENTION

PROBLEM TO BE SOLVED BY THE INVENTION

Conventional image extracting apparatuses extract, as a scene change image from a sequence of captured images, an image of a scene where the content of image is largely changed. In this manner, the scene change image is extracted in many cases based on the change of images, and not based on the change of the contents of the scene. Therefore, the contents captured in a scene change image itself or subsequent images may not be grasped from the scene change image, in other words, scene change images may include an image which is not appropriate as a representative image of the contents of the scene. In this case, there is a problem in that the user may not grasp the contents of the sequence of images in some parts only by checking the scene change image.
In addition, the conventional image processing apparatus extracts a plurality of images between scene change frames, i.e., scene change images so that the user can definitely grasp the contents of the sequence of images. In this case, there is a problem in that the number of images extracted by the image processing apparatus increases and thus the user takes a long time to view these images.
The present invention has been achieved in view of the above problems, and an object of the invention is to provide an image extracting apparatus that can extract an image by which a user easily grasps the contents of the sequence of images in a short viewing time, an image extracting program therefor, and an image extracting method therefor.

MEANS FOR SOLVING PROBLEM

To solve the problems as described above and to achieve an object, an image extracting apparatus according to the invention includes a scene-change-image extracting unit that extracts a scene change image that is an image located at a position at which a scene is changed from an image sequence acquired in time series, a comparing unit that compares the scene change image with an adjacent image that is acquired at a time adjacent to the scene change image within a predetermined time-series range, and a summary image extracting unit that extracts one of the scene change image and the adjacent image as a summary image by using a result of comparison by the comparing unit.
Further, an image extracting program according to the invention causes a computer to perform: a scene change image extracting step of extracting a scene change image that is an image located at a position at which a scene is changed from an image sequence acquired in time series; a comparing step of comparing the scene change image with an adjacent image that is acquired at a time adjacent to the scene change image within a predetermined time-series range; and a summary image extracting step of extracting one of the scene change image and the adjacent image as a summary image by using a result of comparison in the comparing step.

Still further, an image extracting method according to the invention includes a scene change image extracting step of extracting a scene change image that is an image located at a position at which a scene is changed from an image sequence acquired in time series, a comparing step of comparing the scene change image with an adjacent image that is acquired at a time adjacent to the scene change image within a predetermined time-series range, and a summary image extracting step of extracting one of the scene change image and the adjacent image as a summary image by using a result of comparison in the comparing step.

EFFECT OF THE INVENTION

When a scene change image is compared with an adjacent image thereof and the adjacent image is an image by which a user easily grasps the contents of the sequence of images, because the image extracting apparatus according to the present invention can extract the adjacent image instead of the scene change image, an image by which the user easily grasps the contents of the sequence of images in a short viewing time can be extracted.

BRIEF DESCRIPTION OF DRAWINGS

FIG. 1 is a block diagram illustrating the configuration of an image extracting apparatus according to a first embodiment of the present invention.

FIG. 2 is a flowchart illustrating an image extraction processing procedure performed by the image extracting apparatus illustrated in FIG. 1.

FIG. 3 is a flowchart illustrating the processing procedure of a summary image extraction process illustrated in FIG. 2.

FIG. 4 is a schematic diagram illustrating an image extraction process performed by the image extracting apparatus illustrated in FIG. 1.

FIG. 5 is a diagram illustrating a specific example of a scene change image and an adjacent image.

FIG. 6 is a flowchart illustrating the processing procedure of the summary image extraction process illustrated in FIG. 2.

FIG. 7 is a flowchart illustrating the processing procedure of the summary image extraction process illustrated in FIG. 2.

FIG. 8 is a diagram illustrating an example of a predetermined range on an image.

FIG. 9 is a block diagram illustrating the configuration of an image extracting apparatus according to a second embodiment of the present invention.

FIG. 10 is a flowchart illustrating the processing procedure of the summary image extraction process illustrated in FIG. 2.

FIG. 11 is a diagram illustrating the configuration of an intra-subject information acquiring system according to an example of the present invention.

FIG. 12 is a diagram illustrating an example of an extraction condition of a main subject according to the example of the present invention.

FIG. 13 is a block diagram illustrating the configuration of an image extracting apparatus according to a third embodiment of the present invention.

FIG. 14 is a flowchart illustrating an image extraction processing procedure performed by the image extracting apparatus illustrated in FIG. 13.

FIG. 15 is a schematic diagram illustrating an image extraction process performed by the image extracting apparatus illustrated in FIG. 13.

FIG. 16 is a diagram illustrating a display example of an image.

FIG. 17 is a diagram illustrating a display example of an image.

FIG. 18 is a flowchart illustrating the processing procedure of the summary image extraction process illustrated in FIG. 2.

FIG. 19 is a block diagram illustrating the configuration of an image extracting apparatus according to a fifth embodiment of the present invention.

FIG. 20 is a flowchart illustrating the processing procedure of the summary image extraction process illustrated in FIG. 2.

EXPLANATIONS OF LETTERS OR NUMERALS

| | |
|---|---|
| 1, 100, 200, 300 | Control unit |
| 2 | Storage unit |
| 3 | Display unit |
| 3a | Display screen |
| 3b | Image display surface |
| 4 | Input unit |
| 5 | Capsule endoscope |
| 6 | Receiving apparatus |
| 6a, 6b, 6c, 6d, 6e, 6f, 6g, 6h | Receiving antenna |
| 10, 20, 30-2, 30-i, 40, 41 | Image sequence |
| 11 | Image reading unit |
| 12 | Scene-change-image extracting unit |
| 13, 15, 17 | Summary image extracting unit |
| 14 | Number-of-extraction-images setting unit |
| 16 | Selection information acquiring unit |
| 10-1 to 10-N | Image |
| 20-1 to 20-n | Scene change image |
| 30-i-1 to 30-i-n' | Adjacent image |
| 51 | Suspend button |
| 52 | Play button |
| 53 | Reverse play button |
| 54 | Forward-direction frame advance button |
| 55 | Backward-direction frame advance button |
| 56 | Selection button |
| 101, 102, 103, 104 | Object |
| 131, 152 | Main subject extracting unit |
| 132, 153, 171 | Comparing unit |
| 133, 154, 172 | Extracting unit |
| 151 | Observation target specifying unit |
| H | Subject |
| S | Predetermined range |

BEST MODE(S) FOR CARRYING OUT THE INVENTION

Exemplary embodiments of an image extracting apparatus, an image extracting program, and an image extracting method according to the present invention will be explained below in detail with reference to the accompanying drawings. The present invention is not limited to the embodiments explained below. In the description of drawings, the same or similar parts have the same symbols.

(First Embodiment)

FIG. 1 is a block diagram illustrating the configuration of an image extracting apparatus according to the first embodiment of the present invention. The image extracting apparatus illustrated in FIG. 1 includes a control unit 1 that has a calculation function and a control function, a storage unit 2 that stores therein various types of information including image information of consecutive image sequences, a display unit 3 that displays information including images, and an input unit 4 such as a keyboard or a mouse. The image extracting apparatus is realized by a computer that includes a CPU, a ROM, a RAM, and the like.

The control unit 1 includes an image reading unit 11, a scene-change-image extracting unit 12, a summary image extracting unit 13, and a number-of-extraction-images setting unit 14. The image reading unit 11 reads temporally consecutive images within an image sequence that is stored in the storage unit 2. The scene-change-image extracting unit 12 extracts a scene change image, in other words, an image located at the position at which a scene in the image sequence read by the image reading unit 11 is changed. The summary image extracting unit 13 compares the scene change image with an adjacent image, in other words, an image acquired at a time point adjacent to the scene change image in a predetermined time-series range and extracts one of the scene change image and the adjacent image as a summary image indicative of the content of the image sequence by using the comparison result. The number-of-extraction-images setting unit 14 sets the number of extraction images based on the number of images of the image sequence.

The scene-change-image extracting unit 12 computes feature data for each image by using the image information read by the image reading unit 11 and computes a change amount of feature data between predetermined images, to extract an image in which the change amount of feature data is large as a scene change image. Next, it will be explained about a scene-change-image extraction process performed by the scene-change-image extracting unit 12.

First, it is assumed that the number of feature data of an image in an image sequence is M (M is two or more as an integer number) and the number of images in the image sequence is N (N is two or more as an integer number). Moreover, it is assumed that the image information of the image in the image sequence is $I_1$ to IN in order of captured time. When a change amount of p-th feature data given to a q-th image is denoted as $\Delta F_{qp}$, a change amount of feature data given to an image of an image sequence 10 is indicated as a change-amount matrix ΔF expressed by the following Equation (1).

$$\Delta F = \begin{pmatrix} \Delta F_{11} & \Delta F_{12} \cdots & \Delta F_{1p} \cdots & \Delta F_{1M} \\ \Delta F_{21} & \ddots & \vdots & \\ \vdots & & \ddots & \\ \Delta F_{q1} & \cdots\cdots & \Delta F_{qp} & \\ \vdots & & & \ddots \\ \Delta F_{N1} & & & \Delta F_{NM} \end{pmatrix} \quad (1)$$

In this method, different types of a plurality of feature data is used and the sizes of feature data are not unified. Therefore, when simply constituting vectors of which components are change amounts of each feature data and performing a threshold process on the magnitude of vector, the influence of feature data having a relatively high value becomes dominant and the influence of feature data having a relatively small value is not reflected. In the first embodiment, a normalization process is performed on the change amount of the plurality of feature data. Vectors of change amounts of feature data of which components are values obtained by the normalization process are defined as change-amount vectors for each image.

Specifically, the change-amount vector $\vec{f}_q$ of an image 10-q is expressed by the following Equation (2).

$$\vec{f}_q = \sum_{j=1}^{M} w_j \left\{ \kappa_j \cdot (\Delta F_{qj} - \Delta \overline{F}_j) \cdot \vec{i}_j \right\} \quad (2)$$

In this case, a unit vector $i_q$(p=1, 2, ..., M) of a dimension corresponding to each feature data direction satisfies ∀p,p' (p≠p', p=1, 2, ..., M), $i_p \perp i_{p'}$. In the equation (2), weight coefficients for feature data are $w_1$ to $w_M$. Furthermore, an (image) average $\overline{\Delta Fp}$ of feature data and a normalization coefficient $\kappa_p$ are respectively expressed by the following Equations (3) and (4).

$$\Delta \overline{F}_j = \frac{1}{N} \sum_{j=1}^{N} \Delta F_{qj} \quad (3)$$

$$\kappa_j = \left\{ \frac{1}{N} \sum_{j=1}^{N} (\Delta F_{ip} - \Delta \overline{F}_p)^2 \right\}^{-\frac{1}{2}} = \frac{1}{\sigma_p} = \frac{1}{\sqrt{\nu_p}} \quad (4)$$

In Equation (4), $\sigma_p$ indicates standard deviation and $\nu_p$ indicates variance.

When a change-amount vector of each image is given by Equation (2), an integrated change amount Simq obtained by combining and integrating the change amounts of the plurality of feature data for each image is given by the following Equation (5).

$$Sim_q = \| \vec{f}_q \| \quad (5)$$

The integrated change amount Simq indicates a degree of similarity between predetermined images. The degree of similarity between predetermined images is high when the integrated change amount Simq is small. Therefore, the scene-change-image extracting unit 12 sequentially extracts the predetermined number of images from an image having a large integrated change amount $Sim_q$ as a scene change image. In this case, the number of scene change images extracted by the scene-change-image extracting unit 12 is previously set by the number-of-extraction-images setting unit 14. However, the number of scene change images can be input into the number-of-extraction-images setting unit 14 via the input unit 4 by a user.

The summary image extracting unit 13 includes a main subject extracting unit 131, a comparing unit 132, and an extracting unit 133. The main subject extracting unit 131 extracts a main subject captured in the scene change image and the adjacent image by using the image information of the scene change image and the adjacent image. The comparing unit 132 compares the image information of the scene change image and the image information of the adjacent image. The extracting unit 133 extracts as a summary image one of the scene change image or the adjacent image corresponding to the scene change image by using a result of comparison by the comparing unit 132.

The user previously inputs the type of the main subject and an extraction condition thereof into the main subject extracting unit 131 via the input unit 4. The main subject is a target object on which the user focuses when viewing the image, for example, a person. The main subject extracting unit 131 receives the designated type of the main subject from the user, reads an extraction condition corresponding to the main subject designated by the user from the extraction condition of the main subject previously stored in the storage unit 2, and extracts a main subject captured in an image by using the read extraction condition. The extraction method of a main subject can be performed by various known techniques. For example, there is a method for extracting an area of an image by using edge extraction or texture analysis, computing feature data (color, shape, size, frequency, and the like) for each area, and comparing the computed feature data for each area with the feature data of the main subject to be extracted, in order to extract a main subject. Alternatively, there is also a method for performing a template matching process of extracted main subjects to extract a main subject. Furthermore, there is a method for extracting a main subject by using a known technique for recognizing an object.

The CPU that is included in the image extracting apparatus and has these units as described above reads an image processing program for performing an image extraction process according to the first embodiment from the storage unit 2 and performs arithmetic processing related to the image extraction process. The image processing program according to the first embodiment can be recorded in a computer-readable recording medium such as a flexible disk, CD-ROM, DVD-ROM, or a flash memory to be distributed widely. Therefore, the image extracting apparatus according to the first embodiment can include an auxiliary storage device that can read any of the various types of recording media.

FIG. 2 is a flowchart illustrating an image extraction processing procedure according to the first embodiment. First, the image reading unit 11 reads information such as the total number of images constituting an image sequence or the size of image from the storage unit 2 (Step S101). After that, the scene-change-image extracting unit 12 extracts a scene change image in the image sequence by using the image information read by the image reading unit 11 (Step S102). After that, the summary image extracting unit 13 compares the scene change image with an adjacent image corresponding to this scene change image for each scene change image and extracts any one of the scene change image and the adjacent image as a summary image (Step S103). Then, the summary image is output to the display unit 3 to be displayed thereon (Step S104).

Next, it will be explained about a specific processing procedure of a summary image extraction process (Step S103). FIG. 3 is a flowchart illustrating the procedure of the summary image extraction process for comparing a scene change image and an adjacent image corresponding to the scene change image for each scene change image and extracting any one of the scene change image and the adjacent image as a summary image. First, the main subject extracting unit 131 extracts a main subject captured in the scene change image and the adjacent image for each scene change image (Step S201). After that, the comparing unit 132 determines whether the main subject is extracted from the scene change image, in other words, whether the main subject is captured in the scene change image (Step S202). When the main subject is captured in the scene change image (Step S202: Yes), the comparing unit 132 computes a square measure by which the main subject captured in the scene change image occupies its image and a square measure by which the main subject captured in the adjacent image occupies its image (Step S203). Then, the comparing unit 132 compares the square measure of the main subject occupied on the scene change image and the square measure of the main subject occupied on the adjacent image (Step S204). When the main subject on the scene change image is the largest (Step S204: Yes), the extracting unit 133 extracts the scene change image as a summary image (Step S205).

On the other hand, when the square measure of the main subject occupied on the scene change image is smaller than the square measure of the main subject occupied on the adjacent image (Step S204: No), the extracting unit 133 extracts the adjacent image in which the square measure of the main subject is the largest as a summary image instead of the scene change image (Step S206).

The process of Step S204 is performed on an adjacent image, in which the same main subject as that captured in the scene change image is captured, as well as the scene change image. Therefore, the comparing unit 132 determines whether the main subject on the scene change image is equal to the main subject on the adjacent image. When the main subject on the adjacent image is not equal to the main subject on the scene change image, the adjacent image is not used for comparison.

When the main subject is not captured in the scene change image (Step S202: No), the comparing unit 132 determines whether the main subject is extracted from the adjacent image, in other words, whether the main subject is captured in the adjacent image (Step S207). When the main subject is captured in the adjacent image (Step S207: Yes), the comparing unit 132 computes the square measure of the main subject on each adjacent image (Step S208). After that, the process moves to Step S206. The extracting unit 133 extracts the adjacent image in which the square measure of the main subject is the largest as a summary image. On the other hand, when the main subject is not captured in the adjacent image (Step S207: No), the extracting unit 133 extracts the scene change image as a summary image (Step S209).

The summary image extracting unit 13 performs the process for each scene change image. After performing the process on all scene change images, the summary image extracting unit 13 terminates the summary image extraction process (Step S210).

In this manner, the image extracting apparatus according to the first embodiment compares the square measures of the main subjects on the scene change image and the adjacent image for each scene change image by using the image extraction process illustrated in FIGS. 2 and 3, and extracts as a summary image an image in which the square measure of the main subject is the largest, namely an image in which the main subject is most largely captured.

Next, it will be concretely explained about the image extraction process performed by the image extracting apparatus according to the first embodiment with reference to FIG. 4. FIG. 4 is a schematic diagram illustrating a process for extracting a scene change image from the temporally consecutive image sequence 10 and extracting an adjacent image of each scene change image to extract a summary image. The temporally consecutive image sequence 10 has N images 10-1 to 10-N that are adjacent to each other at time intervals $\Delta t$. By using the process of Step S102 illustrated in FIG. 2, the scene-change-image extracting unit 12 extracts a scene-change-image sequence 20 (20-1 to 20-n) having n ($n<N$) images from the image sequence 10. As a result, the images 10-1 to 10-N seems to be divided into n sections by the scene change images 20-1 to 20-n.

After that, by using the process illustrated in FIG. 3, the summary image extracting unit 13 extracts a summary-image sequence 40 by using any one of the scene change image and the adjacent image as a summary image. As illustrated in FIG. 4, the summary image extracting unit 13 compares, for example, the scene change image 20-i and adjacent images 30-i-1 to 30-i-n' and extracts the adjacent image 30-i-i as a summary image. Moreover, the summary image extracting unit 13 extracts as a summary image the scene change images 20-1, 20-(i-1), and 20-n and adjacent images 30-2-j and 30-(i+1)-k.

In FIG. 4, the adjacent images are, among images in the image sequence, images acquired before and after the time at which the scene change image is acquired. However, the adjacent image can be an image acquired before the time at which the scene change image is acquired or an image acquired after the time at which the scene change image is acquired.

Next, it will be explained about a specific example of the scene change image, the adjacent image, and the summary image with reference to FIG. 5. FIG. 5 is a diagram illustrating a part of an image in the image sequence 10. In FIG. 5, objects 101, 102, 103, and 104 indicating a part or the whole of a star-shaped object are respectively captured in four images 10-i, 10-(i+1), 10-(i+2), and 10-(i+3) that are adjacent to each other at time intervals $\Delta t$. These four objects 101, 102, 103, and 104 are the same object. In FIG. 5, the star-shaped object moves to the right side in a horizontal direction in the same visual field. In the image 10-i, a one-third part of the star-shaped object appears in the image. In the image 10-(i+1), a half part of the star-shaped object appears in the image. In the image 10-(i+2), the substantially whole part of the star-shaped object appear in the image. In the image 10-(i+3), the whole of the star-shaped object appears in the image.

The scene-change-image extracting unit 12 extracts, among the images in the image sequence 10, an image in which a change amount of feature data between predetermined images is large as a scene change image. The feature data includes the number of images or color of a subject, a square measure of a subject in an image, and the like. When the scene-change-image extracting unit 12 extracts a scene change image by using a change amount of feature data between images that are separated from each other by the time interval $\Delta t$, the change between the image 10-(i+1) and the image 10-(i+2) is the largest in the images illustrated in FIG. 5. Therefore, the scene-change-image extracting unit 12 extracts the image 10-(i+2) as a scene change image.

After that, the summary image extracting unit 13 compares the main subjects of the image 10-(i+2) that is a scene change image and the images 10-(i+1) and 10-(i+3) that are adjacent images, in other words, the square measures of the objects 102, 103, and 104, and extracts the image 10-(i+3) of which the square measure of the object is large as a summary image.

The image extracting apparatus according to the first embodiment compares the square measure of the main subject on the scene change image with the square measure of the main subject on the adjacent image and extracts an image of which the square measure of the main subject is large as a summary image. Therefore, it is possible to extract an image in which the state of the main subject can be easily observed, in other words, an image by which the contents of the image sequence can be easily grasped. Moreover, because the number of summary images is the same as the number of scene change images, the user can grasp the contents of the image sequence in a short viewing time.

(First Alternative Example)

In the first embodiment, an image in which a main subject is most largely captured among a scene change image and adjacent images is extracted as a summary image. However, in the first alternative example, an image in which a main subject is most visually in focus among a scene change image and adjacent images is extracted as a summary image.

In other words, similarly to the summary image extraction process illustrated in FIG. 3, in the summary image extraction process of Step S103 according to the first embodiment as illustrated in FIG. 6, the main subject extracting unit 131 extracts the main subjects of a scene change image and adjacent images for each scene change image (Step S301), and the comparing unit 132 determines whether the main subject is captured in the scene change image (Step S302). When the main subject is captured in the scene change image (Step S302: Yes), the comparing unit 132 detects the distributions of spatial frequencies of the main subjects on the scene change image and the adjacent images by using the image information of the scene change image and the adjacent image (Step S303). After that, the comparing unit 132 compares the in-focus degrees of the main subjects of the scene change image and the adjacent images by using the distributions of spatial frequencies of the main subjects (Step S304). When the main subject on the scene change image is most visually in focus (Step S304: Yes), the extracting unit 133 extracts the scene change image as a summary image (Step S305).

On the other hand, when the main subject on the adjacent image is visually in focus compared with the main subject on the scene change image (Step S304: No), the extracting unit 133 extracts as a summary image an adjacent image in which the main subject is most visually in focus (Step S306).

In this case, the process of Step S304 is performed on the scene change image and the adjacent images in which the same main subjects as the main subject on the scene change image are captured. Therefore, the comparing unit 132 determines whether the main subject on the scene change image is equal to the main subject on the adjacent image. When the main subject on the adjacent image is not equal to the main subject on the scene change image, the adjacent image is not used for comparison.

When the main subject is not captured in the scene change image (Step S302: No), the comparing unit 132 determines whether the main subject is captured in one of the adjacent images (Step S307). When the main subject is captured in the adjacent image (Step S307: Yes), the comparing unit 132 detects the spatial frequency distribution of the main subject on the adjacent image (Step S308). After that, the process moves to Step S306. The extracting unit 133 extracts as a summary image an adjacent image in which the main subject is most visually in focus. On the other hand, when the main subject is not also captured in the adjacent image (Step S307: No), the extracting unit 133 extracts the scene change image as a summary image (Step S309).

The summary image extracting unit 13 performs the process for each scene change image. After performing the process on all scene change images, the summary image extracting unit 13 terminates the summary image extraction process (Step S310).

In this case, an image in which the main subject is visually in focus is an image in which a high-frequency component is large in the spatial frequency distribution of the part indicative of the main subject. When the same object is used as a main subject, the main subject on an image is displayed as finely as the high-frequency component is large. The method for extracting an image in which the main subject is visually in focus includes a method for extracting the edges (outlines) of the main subjects and comparing edge intensities and a method for comparing contrasts of the color of main subject and the color of background on the image in addition to the method for analyzing the distribution of spatial frequency. In these cases, the extracting unit 133 extracts an image in which an edge intensity is high or an image in which a contrast is high as an image in which the main subject is visually in focus.

The image extracting apparatus according to the first alternative example compares the in-focus degree of the main subject on the scene change image with the in-focus degree of the main subject on the adjacent image and extracts as a summary image an image in which the main subject is most visually in focus. Therefore, an image in which the main subject is clearly captured, in other words, an image that can easily grasp the contents of the image sequence can be extracted.

(Second Alternative Example)

In the second alternative example, among a scene change image and adjacent images, an image in which a main subject is captured in a predetermined range thereon is extracted as a summary image.

In other words, similarly to the summary image extraction process illustrated in FIG. 3, in the summary image extraction process of Step S103 illustrated in FIG. 2, as illustrated in FIG. 7, the main subject extracting unit 131 extracts the main subjects of a scene change image and adjacent images for each scene change image (Step S401). The comparing unit 132 determines whether the main subject is captured in the scene change image (Step S402). When the main subject is captured in the scene change image (Step S402: Yes), the comparing unit 132 detects the positions of the main subjects on the scene change image and the adjacent images (Step S403). After that, the comparing unit 132 determines whether the main subject captured in the scene change image falls within a predetermined range S thereon (Step S404). When the main subject captured in the scene change image falls within the predetermined range S thereon (Step S404: Yes), the extracting unit 133 extracts the scene change image as a summary image (Step S405).

On the other hand, when the main subject captured in the scene change image does not fall within the predetermined range S thereon (Step S404: No), the comparing unit 132 determines whether the main subject captured in the adjacent image falls within the predetermined range S thereon (Step S406). When the main subject captured in the adjacent image falls within the predetermined range S thereon (Step S406: Yes), the extracting unit 133 extracts, for example, an image in which the main subject is captured most near the center of the predetermined range S (Step S407). On the other hand, when the main subject captured in the adjacent image does not fall within the predetermined range S thereon (Step S406: No), the process moves to Step S405. The extracting unit 133 extracts the scene change image as a summary image.

The process of Steps S404 and S406 is performed on the scene change image and the adjacent images in which the same main subjects as the main subject on the scene change image are captured. Therefore, the comparing unit 132 determines whether the main subject on the scene change image is equal to the main subject on the adjacent image. When the main subject on the adjacent image is not equal to the main subject on the scene change image, the adjacent image is not used for comparison.

When the main subject is not captured in the scene change image (Step S402: No), the comparing unit 132 determines whether the main subject is captured in the adjacent image (Step S408). When the main subject is captured in the adjacent image (Step S408: Yes), the comparing unit 132 detects the positions of the main subjects on the adjacent images (Step S409). After that, the process moves to Step S406. On the other hand, when the main subject is not also captured in the adjacent image (Step S408: No), the extracting unit 133 extracts the scene change image as a summary image (Step S410).

The summary image extracting unit 13 performs the process for each scene change image. After performing the process on all scene change images, the summary image extracting unit 13 terminates the summary image extraction process (Step S411).

In this case, the predetermined range S on the image indicates, for example, a range corresponding to the inside of a circle of which the center is the center of an image as illustrated in FIG. 8. However, because a main subject may not be easily observed depending on the imaging content of image if the main subject is located near the center of image, the predetermined range can be set at the position other than the center of image. It is assumed that a user sets a predetermined range in accordance with an imaging target. Specifically, the user previously inputs a predetermined range into the comparing unit 132 via the input unit 4.

The image extracting apparatus according to the second alternative example compares the position of the main subject on the scene change image with the position of the main subject on the adjacent image and extracts an image in which the main subject is captured in the predetermined range. Therefore, an image in which a main subject is captured at the position that can be easily observed by the user, in other words, an image in which the contents of the image sequence are precisely shown can be extracted.

Among a scene change image and adjacent images, an image in which a main subject is most visually in focus in the first alternative example and an image in which the position of a main subject is captured within a predetermined range in the second alternative example are respectively extracted as a summary image. However, another configuration can be employed. For example, the image extracting apparatus can have a configuration operable to compare the brightness of the main subject on the scene change image with the brightness of the main subject on the adjacent image and to extract an image of which exposure is reasonable as a summary image. In other words, the image extracting apparatus can employ an extraction method for computing the pixel intensity of subject and extracting an image of which exposure is more appropriate when the pixel intensity is too large and causes overexposure or the pixel intensity is too small and causes underexposure. In this case, a pixel average of a main subject is computed as pixel intensity, for example. Moreover, assuming that the main subject is within the whole image area, the image extracting apparatus can also have a configuration operable to compare the brightness of the scene change image and the brightness of the adjacent image and to extract an image of which exposure is reasonable as a summary image. In this case, the image extracting apparatus computes, for example, a pixel average of the whole image area as pixel intensity to compare the brightness of the scene change image and the brightness of the adjacent image.

Moreover, the image extracting apparatus can extract one of the scene change image and the adjacent image as a summary image by using two or more comparison results among a comparison result between the square measures of the main subjects, a comparison result between the in-focus degrees of the main subjects, a comparison result between the positions of the main subjects on the images, and a comparison result between the brightness of the main subjects (or the whole image areas). In this case, because the image extracting apparatus extracts one of the scene change image and the adjacent image as a summary image by using the plurality of comparison results, an image which can more easily grasp the contents of the image sequence can be extracted.

(Second Embodiment)

In the first embodiment, an extraction condition of a main subject for each image sequence is not changed regardless of the change of an observation target captured in an image in an image sequence. However, in the second embodiment, an extraction condition of a main subject is automatically changed in accordance with an observation target captured in an image.

In other words, as illustrated in FIG. 9, an image extracting apparatus according to the second embodiment includes an observation target specifying unit 151 in a summary image extracting unit 15 of a control unit 100 in addition to the units of the image extracting apparatus of the first embodiment.

An image extraction processing procedure is similar to Steps S101 to S104 of the first embodiment except for the summary image extraction process procedure of Step S103. In other words, as illustrated in FIG. 10, the observation target specifying unit 151 first specifies an observation target captured in a scene change image for each scene change image (Step S501). After that, a main subject extracting unit 152 changes an extraction condition of a main subject in accordance with the observation target (Step S502). Subsequently, the main subject extracting unit 152 extracts the main subjects of the scene change image and the adjacent images by using the changed extraction condition (Step S503). The summary image extraction process (Steps S504 to S512) after that is similar to the summary image extraction process (Steps S202 to S210) illustrated in the first embodiment.

The image extracting apparatus according to the second embodiment changes the extraction condition of a main subject in accordance with an observation target captured in an image. However, an object to be noticed can be surely extracted as a main subject even if the special feature of the object to be noticed is different depending on the observation target. Therefore, the image extracting apparatus according to the second embodiment can extract an image in which an appropriate main subject is captured depending on the observation target, in other words, an image that can easily grasp the contents of the image sequence.

(Example)

The image extracting apparatus according to the present invention can applied to the extraction of an image sequence (image sequence of a celom) captured by a capsule endoscope. FIG. 11 is a typical diagram illustrating an example of an intra-subject information acquiring system including the image extracting apparatus illustrated in the second embodiment. The intra-subject information acquiring system illustrated in FIG. 11 includes a capsule endoscope 5, a receiving apparatus 6, and the portable storage unit 2. The capsule endoscope 5 is introduced into a celom of a subject H and captures images in the celom. The receiving apparatus 6 receives radio signals transmitted by the capsule endoscope 5 and accumulates images included in the received radio signals. The portable storage unit 2 such as a memory card can be attached to or detached from the receiving apparatus 6 and the image extracting apparatus.

The capsule endoscope 5 has an imaging function for capturing intra-celom images of the subject H and a radio communication function for transmitting radio signals including captured images to the outside. More specifically, the capsule endoscope 5 travels inside the celom of the subject H. At the same time, the capsule endoscope 5 captures the intra-celom images of the subject H, for example, at predetermined intervals (around 2 Hz) of around 0.5 seconds and transmits the captured intra-celom images to the receiving apparatus 6 through a predetermined radio wave.

The receiving apparatus 6 is connected to a plurality of receiving antennas 6a to 6h that receives the radio signals transmitted from the capsule endoscope 5. The receiving antennas 6a to 6h are realized by, for example, loop antennas and are dispersed on the body surface of the subject H corresponding to the traveling path of the capsule endoscope 5. The number of receiving antennas can be one or more on the subject H. The number of receiving antennas is not limited to eight as illustrated.

The receiving apparatus 6 receives the radio signals transmitted from the capsule endoscope 5 via the receiving antennas 6a to 6h and acquires image information of the intra-celom images of the subject H on the basis of the received radio signals. The image information acquired by the receiving apparatus 6 is stored in the storage unit 2 attached to the receiving apparatus 6. The storage unit 2 that stores the image information of the intra-celom images of the subject H is inserted into the image extracting apparatus and is used for a process performed in the control unit 100.

In the intra-subject information acquiring system having the above apparatuses, the capsule endoscope 5 takes time for around eight hours to capture approximately 60,000 images to create an intra-celom image sequence while moving inside the celom of the subject H. Therefore, an intra-celom image sequence includes images in which several kinds of organs such as a stomach or a small intestine are captured.

On the other hand, the special features of the lesioned part noticed by a doctor are different depending on an organ. For example, the size of the noticeable lesioned part in a small intestine is smaller than the size of the noticeable lesioned part in a stomach. For this reason, when the size of a lesioned part is set as one of extraction conditions when extracting the lesioned part as a main subject, a lesioned part expected to be extracted as a main subject in a small intestine may not be extracted and a lesioned part expected not to be extracted as a main subject in a stomach may be extracted if the size of the lesioned part has the same lower-limit threshold value regardless of the type of an observation target. On the contrary, whether an observation target is a stomach or not a small intestine is automatically determined and a lower-limit threshold value appropriate to each of the stomach and the small intestine is automatically set. In this way, a lesioned part of the specific size according to each of the stomach and the small intestine can be surely extracted as a main subject and a lesioned part expected not to be extracted as a main subject can be excluded from extraction targets.

Therefore, the image extracting apparatus according to the example extracts a main subject under different extraction conditions for each organ by particularly using the observation target specifying unit 151 and the main subject extracting unit 152. First, the observation target specifying unit 151 specifies an organ captured in each image by using a spatial frequency component of the image. For example, an esophagus or a stomach has a flat shape, in which the surface of a mucous membrane is not uneven, as compared with a small intestine. Conversely, the surface of a small intestine is uneven due to villus and the like. For this reason, a low frequency component is dominant in images in which an esophagus or a stomach is captured and a high frequency component is dominant in images in which a small intestine is captured. The observation target specifying unit 151 determines whether an organ captured in the observation image is an esophagus or a stomach, a small intestine, or a large intestine by using such a property. Specifically, the observation target specifying unit 151 performs an organ determination, for example, by using power spectrum that is obtained by Fourier transform as spatial frequency component information.

By virtue of the difference of the properties of the mucous membrane surface of each organ, organs captured in images have different properties in terms of the height of a correlation between a predetermined pixel of each image and a peripheral pixel thereof, an amount of texture information of each image, a file size of compressed data of each image, or a DCT coefficient computed during decompressing compressed data. Therefore, the observation target specifying unit 151 can perform an organ determination by comparing these properties. Moreover, because the moving speed of the capsule endoscope 5 is different depending on each organ, a change amount of a frequency component has a difference between images consecutive in time series and a change amount of a file size of compressed data between images or a change amount of a DCT coefficient between images has a difference. Therefore, the observation target specifying unit 151 can compare the change amounts between images to perform an organ determination.

Furthermore, the main subject extracting unit 152 reads an extraction condition according to an organ specified by the observation target specifying unit 151 from the storage unit 2 and extracts a noticeable lesioned part in each organ as a main subject by using the extraction condition.

In the extraction condition, as illustrated in FIG. 12, the lower-limit threshold value of the size of the lesioned part expected to be extracted as a main subject is changed depending on organs. In addition, when the imaging distances of images are different even if the images have lesioned parts of the same size, the sizes (square measures) of the lesioned parts occupied in the images are different. Therefore, as illustrated in FIG. 12, the size of a lesioned part on an image expected to be extracted as a main subject is set for each organ in accordance with imaging distances. In this case, an imaging distance is measured by using bounce brightness. The bounce brightness means brightness when light irradiated from an imaging apparatus is reflected by a subject and then is received by the imaging apparatus as observation light.

Because the intra-subject information acquiring system according to the example extracts a lesioned part that is a main subject by using an extraction condition according to an organ that is an observation target, an appropriate main subject can be extracted for each organ. Therefore, a doctor views a summary image in which an appropriate main subject is captured, and thus can grasp the intra-celom state of a patient in a short time and can quickly diagnose the patient.

In the intra-subject information acquiring system according to the example, the lower-limit threshold value of the size of the lesioned part extracted as a main subject is changed for each organ. However, the condition changed depending on organs is not limited to the size of a lesioned part. The change condition can be the color or shape of a lesioned part, the unevenness of a surface.

(Third Embodiment)

In the first embodiment, all scene change images are compared with adjacent images thereof and any one of the images is extracted as a summary image. However, in the third embodiment, only a scene change image selected by a user is compared with adjacent images and any one of the images is extracted as a summary image.

In other words, as illustrated in FIG. 13, an image extracting apparatus according to the third embodiment includes a selection information acquiring unit 16 in a control unit 200 in addition to the units of the image extracting apparatus according to the first embodiment.

Next, it will be explained about an image extraction processing procedure performed in the image extracting apparatus according to the third embodiment with reference to FIG. 14. FIG. 14 is a flowchart illustrating the image extraction processing procedure according to the third embodiment. First, the image reading unit 11 reads image information for images constituting an image sequence from the storage unit 2 (Step S601). The scene-change-image extracting unit 12 extracts a scene change image in the image sequence (Step S602). After that, the control unit 200 outputs the scene change image and displays it on the display unit 3 (Step S603).

After that, the selection information acquiring unit 16 determines whether the scene change image is selected by a user (Step S604). When the selection information acquiring unit 16 acquires selection information showing that the scene change image is selected (Step S604: Yes), the summary image extracting unit 13 performs a comparison process between the scene change image and adjacent images thereof, in other words, a summary image extraction process on only the scene change image selected by the user (Step S605). In Step S605, the same process as the summary image extraction process (Steps S201 to S210) illustrated in FIG. 3 is performed. After that, the control unit 200 changes the scene change image displayed on the display unit 3 into a summary image (Step S606). Specifically, the control unit 200 displays an adjacent image instead of the displayed scene change image when the adjacent image is extracted as a summary image corresponding to the scene change image selected by the user.

On the other hand, the summary image extracting unit 13 displays the scene change image without performing the summary image extraction process when the selection information showing that the scene change image is selected is not acquired (Step S604: No).

Next, it will be specifically explained about an image extraction process performed by the image extracting apparatus according to the third embodiment with reference to FIG. 15. FIG. 15 is a schematic diagram illustrating a process for extracting a scene change image from a temporally consecutive image sequence and extracting a summary image corresponding to the scene change image selected by a user. The scene-change-image extracting unit 12 extracts the scene change images 20-1 to 20-n from the image sequence 10 by using the process of Step S602. After that, the summary image extracting unit 13 extracts a summary-image sequence 41. Specifically, the summary image extracting unit 13 compares the scene change image 20-2 with the adjacent images 30-2-1 to 30-2-n" corresponding to the scene change image 20-2 to extract the scene change image 20-2 as a summary image and compares the scene change image 20-i with the adjacent images 30-i-1 to 30-i-n' corresponding to the scene change image 20-i to extract the adjacent image 30-i-i as a summary image, by using the process of Step S605. After that, the control unit 200 does not change the scene change image 20-2 into another image and displays the adjacent image 30-i-i on the display unit 3 instead of the scene change image 20-i.

A display example of the scene change image is illustrated in FIGS. 16 and 17. FIG. 16 is a diagram illustrating a display screen 3a of the display unit 3 on which the scene change images are displayed in a thumbnail manner. The selection information acquiring unit 16 inputs the selection information of the scene change image into the summary image extracting unit 13 by using the scene change image clicked by the user as the scene change image selected by the user.

FIG. 17 is a diagram illustrating an example of a so-called moving-image play display of sequentially displaying scene change images on an image display surface 3b of the display screen 3a in time sequence. When the user selects a selection button 56 during displaying the scene change images for play, the selection information acquiring unit 16 inputs the selection information into the summary image extracting unit 13 by using the scene change image displayed during the selection of the selection button 56 as the scene change image selected by the user. As illustrated in FIG. 17, the display screen 3a includes a suspend button 51, a play button 52, a reverse play button 53, a forward-direction frame advance button 54, and a backward-direction frame advance button 55, in addition to the selection button. The suspend button 51 is a button that inputs an instruction for suspending a moving-image display into the control unit 200. The play button 52 is a button that inputs an instruction for displaying images in time sequence into the control unit 200. The reverse play button 53 is a button that inputs an instruction for playing images in reverse time sequence into the control unit 200. The forward-direction frame advance button 54 and the backward-direction frame advance button 55 are buttons that input instructions for delaying play speeds in both play directions and increasing a display time of each image into the control unit 200. The user can adjust a play speed in accordance with the attention degree of each scene change image by using these buttons.

The image extracting apparatus according to the third embodiment performs the summary image extraction process on only the scene change image selected by the user, in other words, only the scene change image in which the captured contents cannot be determined. Therefore, the burden of the process operations of the image processing apparatus is reduced.

(Fourth Embodiment)

In the first embodiment described above, when a main subject is not captured in a scene change image and adjacent images, the scene change image is extracted as a summary image. However, in the fourth embodiment, when a main subject is not captured in a scene change image and adjacent images, an image having the smallest noise among the scene change image and the adjacent images is extracted as a summary image.

In other words, the image extracting apparatus according to the fourth embodiment has the same units as those of the image processing apparatus illustrated in the first embodiment. Moreover, an image extraction processing procedure is similar to Steps S101 to S104 of the first embodiment except for the summary image extraction process procedure of Step S103. In other words, similarly to the summary image extraction process illustrated in FIG. 3, as illustrated in FIG. 18, the main subject extracting unit 131 extracts a main subject on the scene change image and the adjacent images (Step S701). When the main subject is not captured in the scene change image (Step S702: No) and the main subject is not also captured in the adjacent images (Step S707: No), the comparing unit 132 detects the noises of the scene change image and the adjacent images (Step S709). After that, the comparing unit 132 compares the amount of noise of the scene change image with the amount of noise of the adjacent images (Step S710). When the amount of noise of the scene change image is smallest (Step S710: Yes), the extracting unit 133 extracts the scene change image as a summary image (Step S711). On the other hand, when there is an adjacent image having the amount of noise smaller than that of the scene change image (Step S710: No), the extracting unit 133 extracts the adjacent image having the smallest noise as a summary image (Step S712).

The summary image extraction process (Steps S703 to S708 and S713) when the main subject is captured in the scene change image (Step S702: Yes) and when the main subject is not captured in the scene change image and is captured in the adjacent image (Step S707: Yes) is similar to the process of Steps S203 to S208 and S210 illustrated in the summary image extraction process procedure of the first embodiment. Similarly to the first embodiment, the image extracting apparatus according to the fourth embodiment extracts a summary image from one of the scene change image and the adjacent images.

In the fourth embodiment, when a main subject is not captured in a scene change image and adjacent images, a totally clear image that can be easily observed is extracted as a summary image. Therefore, an image of which the imaging contents are easily grasped can be extracted from scenes in which a main subject is not captured, in other words, images that are particularly not a noticeable scene.

(Fifth Embodiment)

Next, it will be explained about the fifth embodiment of the present invention. In the first to fourth embodiments described above, imaging states of main subjects on a scene change image and adjacent images are compared and a summary image is extracted. However, in the fifth embodiment, a summary image is extracted by using the noise amounts of the scene change image and the adjacent images.

In other words, as illustrated in FIG. 19, an image extracting apparatus according to the fifth embodiment includes a summary image extracting unit 17 in a control unit 300 instead of the summary image extracting unit 13 of the image processing apparatus according to the first embodiment. The summary image extracting unit 17 includes a comparing unit 171 and an extracting unit 172. The summary image extracting unit 17 compares the noise amounts of the scene change image and the adjacent images and extracts an image having a small noise amount as a summary image. The other units are similar to those of the image extracting apparatus illustrated in FIG. 1.

An image extraction processing procedure performed in the image extracting apparatus according to the fifth embodiment is similar to the image extraction processing procedure (Steps S101 to S104) performed in the image extracting apparatus according to the first embodiment. However, the summary image extraction process of Step S103 is different. In other words, as illustrated in FIG. 20, the comparing unit 171 detects the noises of the scene change image and the adjacent images for each scene change image (Step S801). After that, the comparing unit 171 compares the amount of noise of the scene change image and the amount of noise of the adjacent images (Step S802). When the noise of the scene change image is the smallest (Step S802: Yes), the extracting unit 172 extracts the scene change image as a summary image (Step S804). On the other hand, when there is an adjacent image having the amount of noise smaller than that of the scene change image (Step S802: No), the adjacent image having the smallest noise is extracted as a scene change image (Step S803). When the process is performed on all scene change images, the summary image extraction process is terminated (Step S805).

The image extracting apparatus according to the fifth embodiment extracts a totally clear image as a summary image irrespective of the presence or absence of a main subject. Therefore, it is possible to extract an image of which the whole can be easily observed, in other words, an image by which the contents of an image sequence are easily grasped without being limited to a main subject.

INDUSTRIAL APPLICABILITY

As described above, the image extracting apparatus, the image extracting program, and the image extracting method according to the present invention are useful for extracting a scene change position and a valid image from a continuously captured image sequence and a moving-image-frame image sequence.

## Claims

1. An image extracting apparatus comprising:

   a scene-change-image extracting unit that extracts a scene change image that is an image located at a position at which a scene is changed from an image sequence acquired in time series;
   a comparing unit that compares the scene change image with an adjacent image that is acquired at a time adjacent to the scene change image within a predetermined time-series range; and
   a summary image extracting unit that extracts one of the scene change image and the adjacent image as a summary image by using a result of comparison by the comparing unit.

2. The image extracting apparatus according to claim 1, comprising a main subject extracting unit that extracts main subjects captured in the scene change image and the adjacent image, wherein
   the comparing unit compares the scene change image and the adjacent image by using square measures of the main subjects on the scene change image and the adjacent image.

3. The image extracting apparatus according to claim 1, comprising a main subject extracting unit that extracts main subjects captured in the scene change image and the adjacent image, wherein
   the comparing unit compares the scene change image and the adjacent image by using spatial frequency components of the main subjects on the scene change image and the adjacent image.

4. The image extracting apparatus according to claim 1, comprising a main subject extracting unit that extracts main subjects captured in the scene change image and the adjacent image, wherein
   the comparing unit compares the scene change image and the adjacent image by using positions of the main subjects on the scene change image and the adjacent image.

5. The image extracting apparatus according to claim 1, comprising a main subject extracting unit that extracts main subjects captured in the scene change image and the adjacent image, wherein
   the comparing unit compares the scene change image and the adjacent image by using pixel intensities of the main subjects on the scene change image and the adjacent image.

6. The image extracting apparatus according to any one of claims 2 to 5, comprising an observation target specifying unit that specifies an observation target captured in the scene change image and the adjacent image, wherein
   the main subject extracting unit changes a main subject extraction condition in accordance with the observation target specified by the observation target specifying unit.

7. The image extracting apparatus according to any one of claims 1 to 6, wherein the image sequence is an intracelom image sequence captured by a capsule endoscope introduced into a celom of a subject.

8. The image extracting apparatus according to any one of claims 2 to 7, wherein the main subject extracting unit extracts a lesioned part captured in the scene change image and the adjacent image as the main subject.

9. The image extracting apparatus according to any one of claims 6 to 8, wherein
   the observation target specifying unit specifies an organ captured in the image as the observation target, and
   the main subject extracting unit changes the main subject extraction condition in accordance with the organ specified by the observation target specifying unit.

**10.** The image extracting apparatus according to any one of claims 2 to 9, wherein the comparing unit compares the scene change image and the adjacent image by using noise amounts of the scene change image and the adjacent image when the main subject extracting unit does not extract the main subjects in the scene change image and the adjacent image.

**11.** The image extracting apparatus according to claim 1, wherein the comparing unit compares the scene change image and the adjacent image by using pixel intensities of the scene change image and the adjacent image.

**12.** The image extracting apparatus according to claim 1, wherein the comparing unit compares the scene change image and the adjacent image by using amounts of noises of the scene change image and the adjacent image.

**13.** The image extracting apparatus according to any one of claims 1 to 12, comprising a number-of-sheets designating unit that designates a number of images extracted by the scene-change-image extracting unit.

**14.** The image extracting apparatus according to claim 1, comprising a main subject extracting unit that extracts main subjects captured in the scene change image and the adjacent image, wherein
the comparing unit compares the scene change image and the adjacent image by using in-focus degrees of the extracted main subjects on the scene change image and the adjacent image.

**15.** The image extracting apparatus according to claim 1, comprising a main subject extracting unit that extracts main subjects captured in the scene change image and the adjacent image, wherein
the comparing unit compares the scene change image and the adjacent image by using information about whether the extracted main subjects on the scene change image and the adjacent image are captured within a predetermined range on the images.

**16.** The image extracting apparatus according to claim 1, comprising a main subject extracting unit that extracts main subjects captured in the scene change image and the adjacent image, wherein
the comparing unit compares the scene change image and the adjacent image by using brightness of the extracted main subjects on the scene change image and the adjacent image.

**17.** An image extracting program causing a computer to perform:

a scene change image extracting step of extracting a scene change image that is an image located at a position at which a scene is changed from an image sequence acquired in time series;
a comparing step of comparing the scene change image with an adjacent image that is acquired at a time adjacent to the scene change image within a predetermined time-series range; and
a summary image extracting step of extracting one of the scene change image and the adjacent image as a summary image by using a result of comparison in the comparing step.

**18.** An image extracting method comprising:

a scene change image extracting step of extracting a scene change image that is an image located at a position at which a scene is changed from an image sequence acquired in time series;
a comparing step of comparing the scene change image with an adjacent image that is acquired at a time adjacent to the scene change image within a predetermined time-series range; and
a summary image extracting step of extracting one of the scene change image and the adjacent image as a summary image by using a result of comparison in the comparing step.

FIG.1

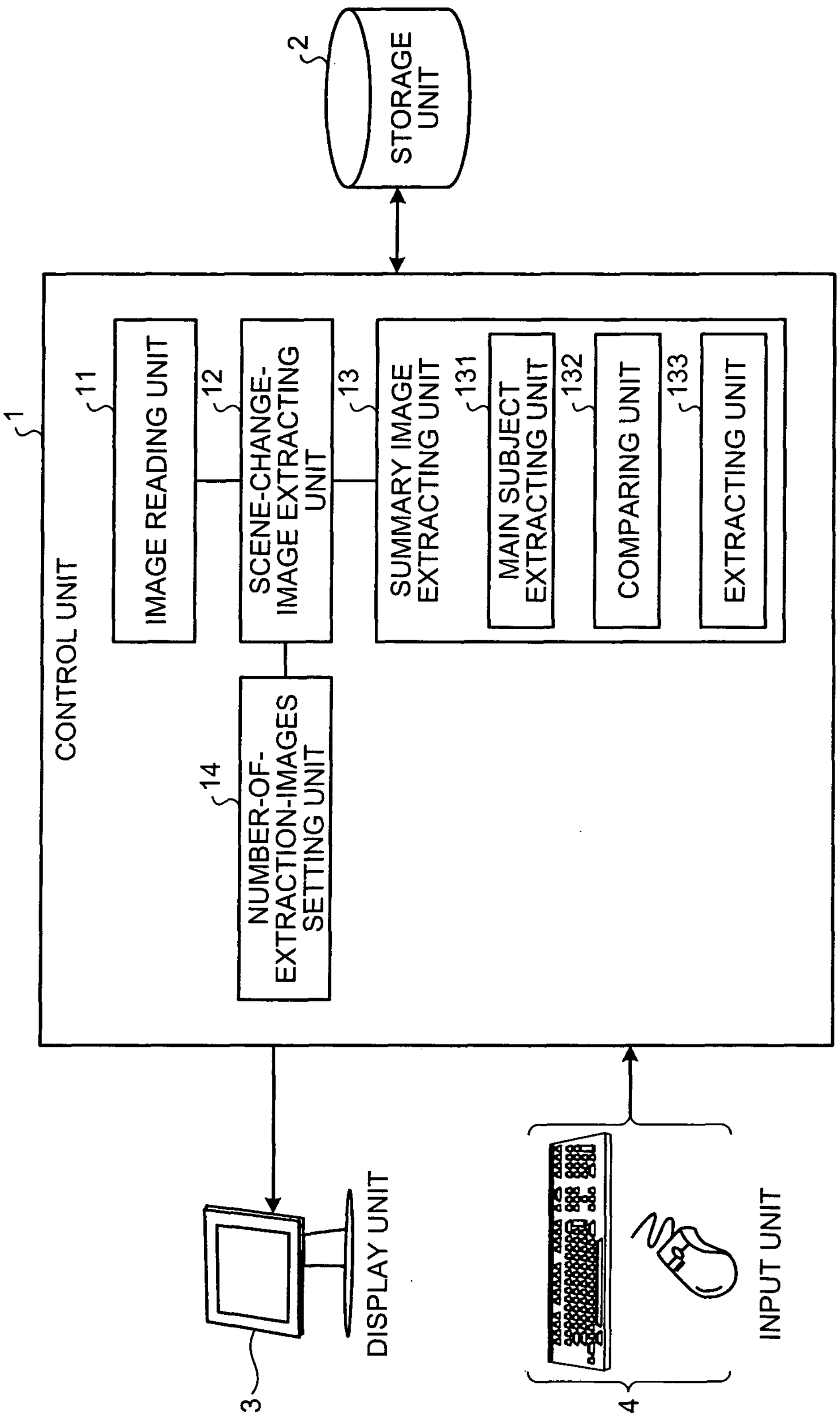
STORAGE UNIT
2
CONTROL UNIT
1
IMAGE READING UNIT
11
SCENE-CHANGE-IMAGE EXTRACTING UNIT
12
SUMMARY IMAGE EXTRACTING UNIT
13
MAIN SUBJECT EXTRACTING UNIT
131
COMPARING UNIT
132
EXTRACTING UNIT
133
NUMBER-OF-EXTRACTION-IMAGES SETTING UNIT
14
DISPLAY UNIT
3
INPUT UNIT
4

FIG.2

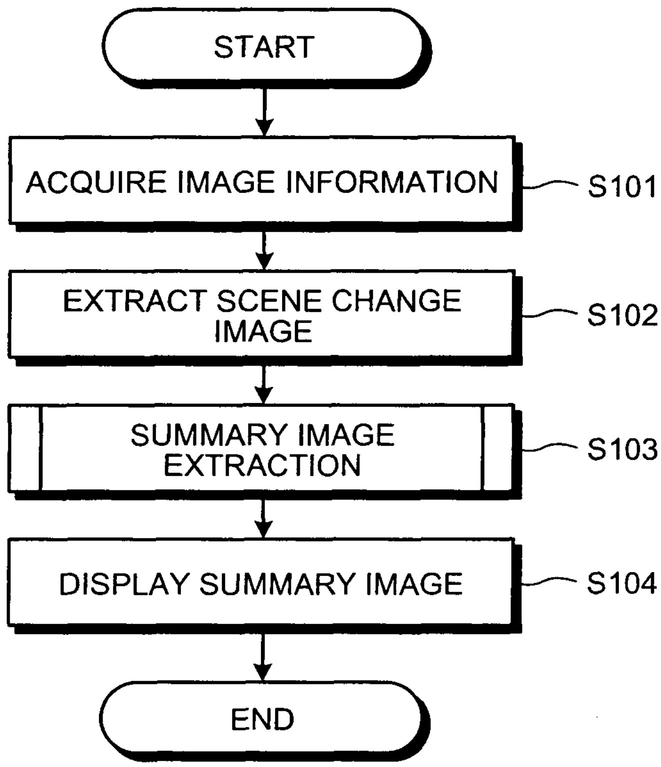
START
ACQUIRE IMAGE INFORMATION
S101
EXTRACT SCENE CHANGE IMAGE
S102
SUMMARY IMAGE EXTRACTION
S103
DISPLAY SUMMARY IMAGE
S104
END

FIG.3

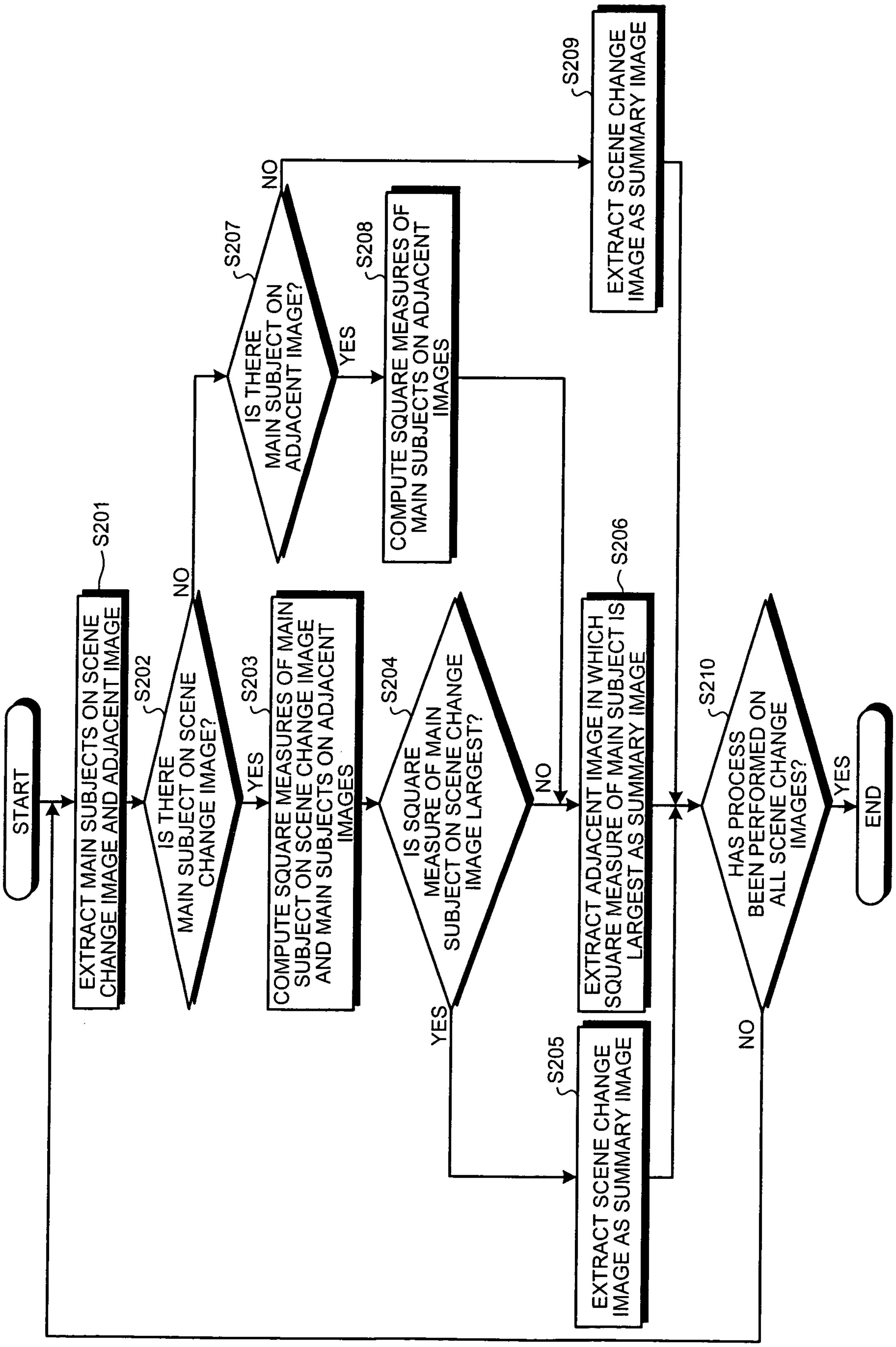
START
EXTRACT MAIN SUBJECTS ON SCENE CHANGE IMAGE AND ADJACENT IMAGE
S201
IS THERE MAIN SUBJECT ON SCENE CHANGE IMAGE?
S202
YES
NO
COMPUTE SQUARE MEASURES OF MAIN SUBJECT ON SCENE CHANGE IMAGE AND MAIN SUBJECTS ON ADJACENT IMAGES
S203
IS SQUARE MEASURE OF MAIN SUBJECT ON SCENE CHANGE IMAGE LARGEST?
S204
YES
NO
EXTRACT SCENE CHANGE IMAGE AS SUMMARY IMAGE
S205
EXTRACT ADJACENT IMAGE IN WHICH SQUARE MEASURE OF MAIN SUBJECT IS LARGEST AS SUMMARY IMAGE
S206
IS THERE MAIN SUBJECT ON ADJACENT IMAGE?
S207
YES
NO
COMPUTE SQUARE MEASURES OF MAIN SUBJECTS ON ADJACENT IMAGES
S208
EXTRACT SCENE CHANGE IMAGE AS SUMMARY IMAGE
S209
HAS PROCESS BEEN PERFORMED ON ALL SCENE CHANGE IMAGES?
S210
YES
NO
END

FIG.4

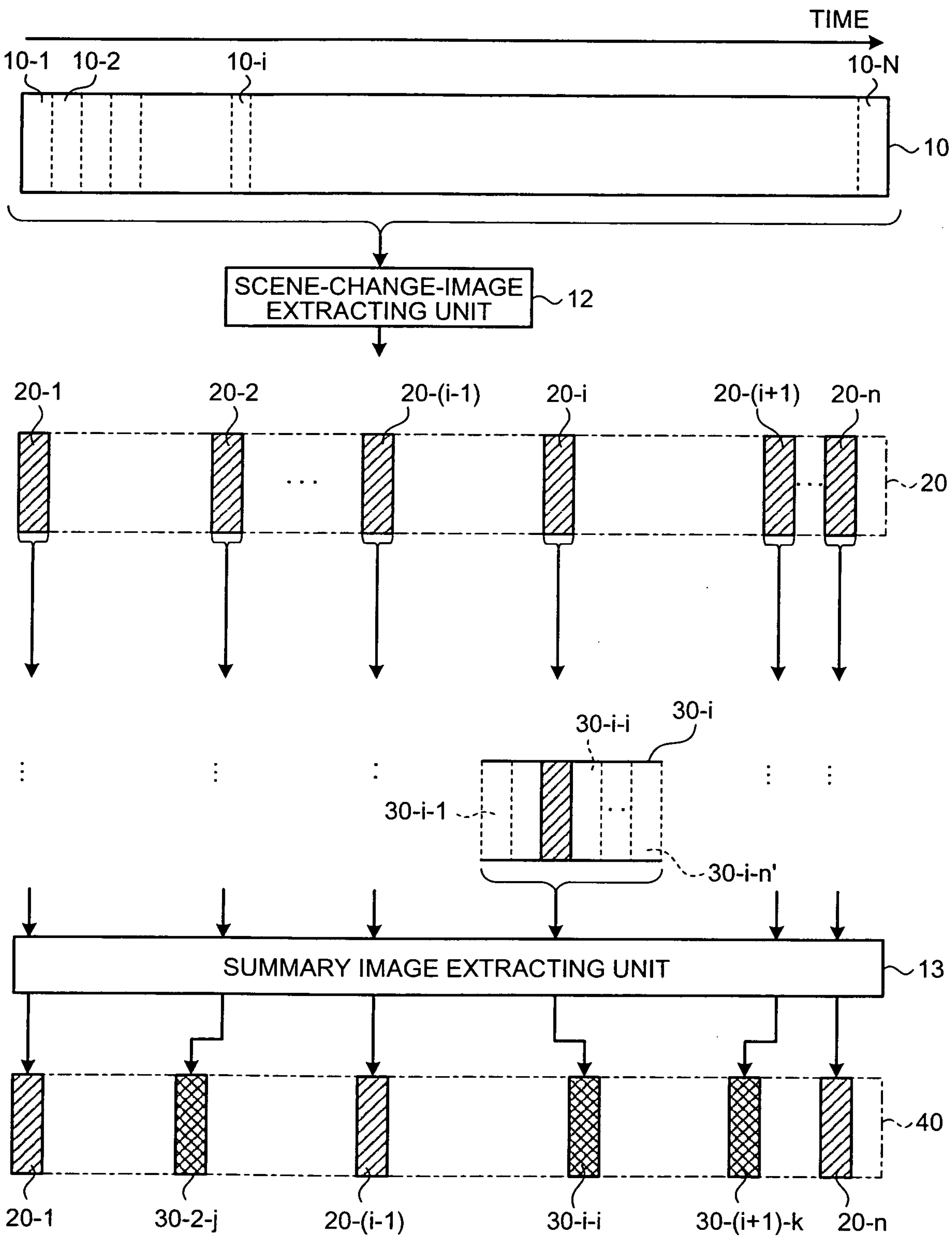
TIME
10-1
10-2
10-i
10-N
10
SCENE-CHANGE-IMAGE EXTRACTING UNIT
12
20-1
20-2
20-(i-1)
20-i
20-(i+1)
20-n
20
30-i-i
30-i
30-i-1
30-i-n'
SUMMARY IMAGE EXTRACTING UNIT
13
40
20-1
30-2-j
20-(i-1)
30-i-i
30-(i+1)-k
20-n

FIG.5

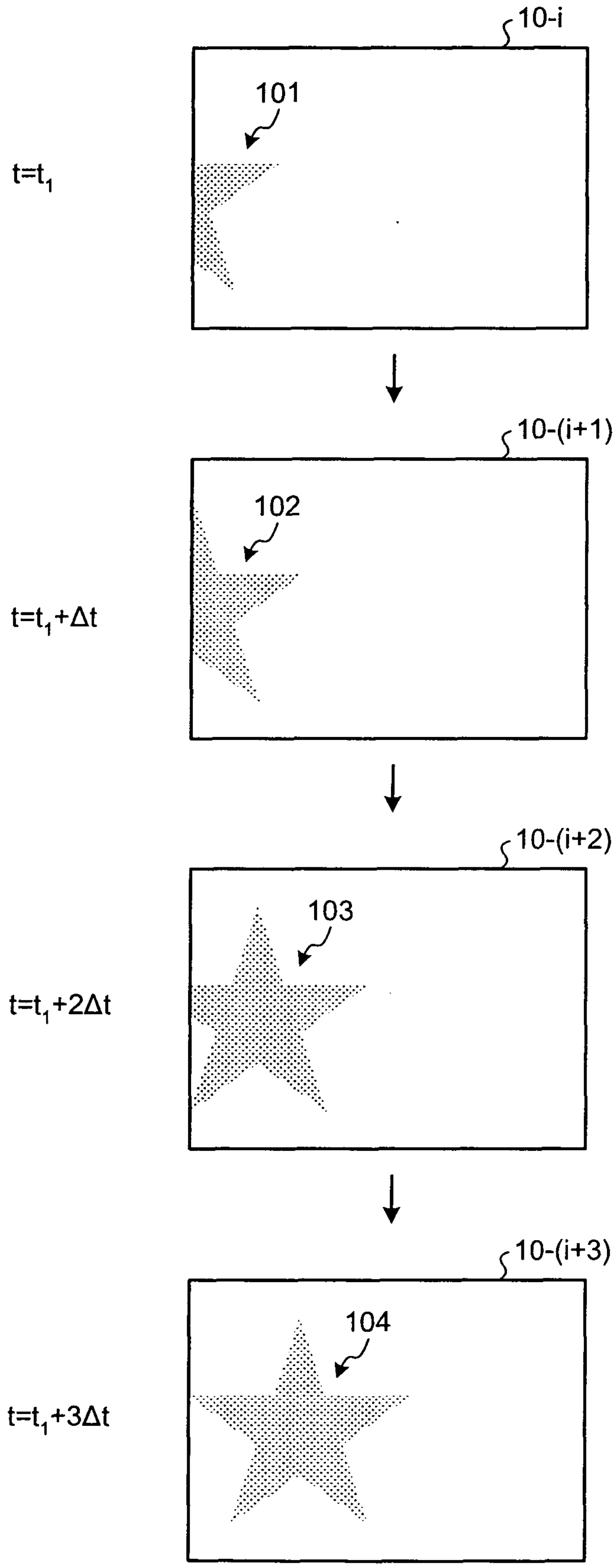

FIG.6

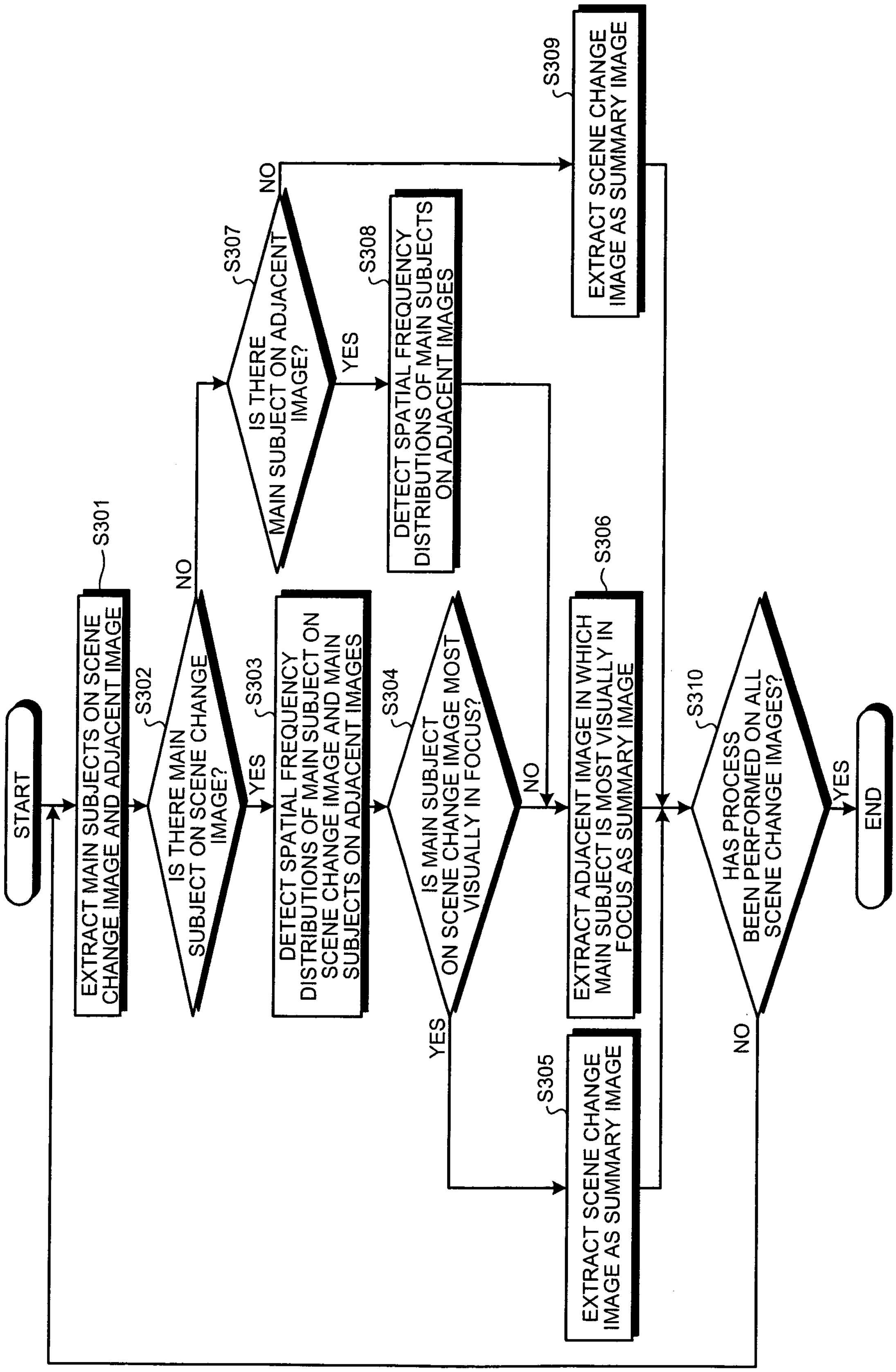
START
EXTRACT MAIN SUBJECTS ON SCENE CHANGE IMAGE AND ADJACENT IMAGE
S301
IS THERE MAIN SUBJECT ON SCENE CHANGE IMAGE?
S302
YES
NO
DETECT SPATIAL FREQUENCY DISTRIBUTIONS OF MAIN SUBJECT ON SCENE CHANGE IMAGE AND MAIN SUBJECTS ON ADJACENT IMAGES
S303
IS MAIN SUBJECT ON SCENE CHANGE IMAGE MOST VISUALLY IN FOCUS?
S304
YES
NO
EXTRACT SCENE CHANGE IMAGE AS SUMMARY IMAGE
S305
EXTRACT ADJACENT IMAGE IN WHICH MAIN SUBJECT IS MOST VISUALLY IN FOCUS AS SUMMARY IMAGE
S306
IS THERE MAIN SUBJECT ON ADJACENT IMAGE?
S307
YES
NO
DETECT SPATIAL FREQUENCY DISTRIBUTIONS OF MAIN SUBJECTS ON ADJACENT IMAGES
S308
EXTRACT SCENE CHANGE IMAGE AS SUMMARY IMAGE
S309
HAS PROCESS BEEN PERFORMED ON ALL SCENE CHANGE IMAGES?
S310
YES
NO
END

FIG.7

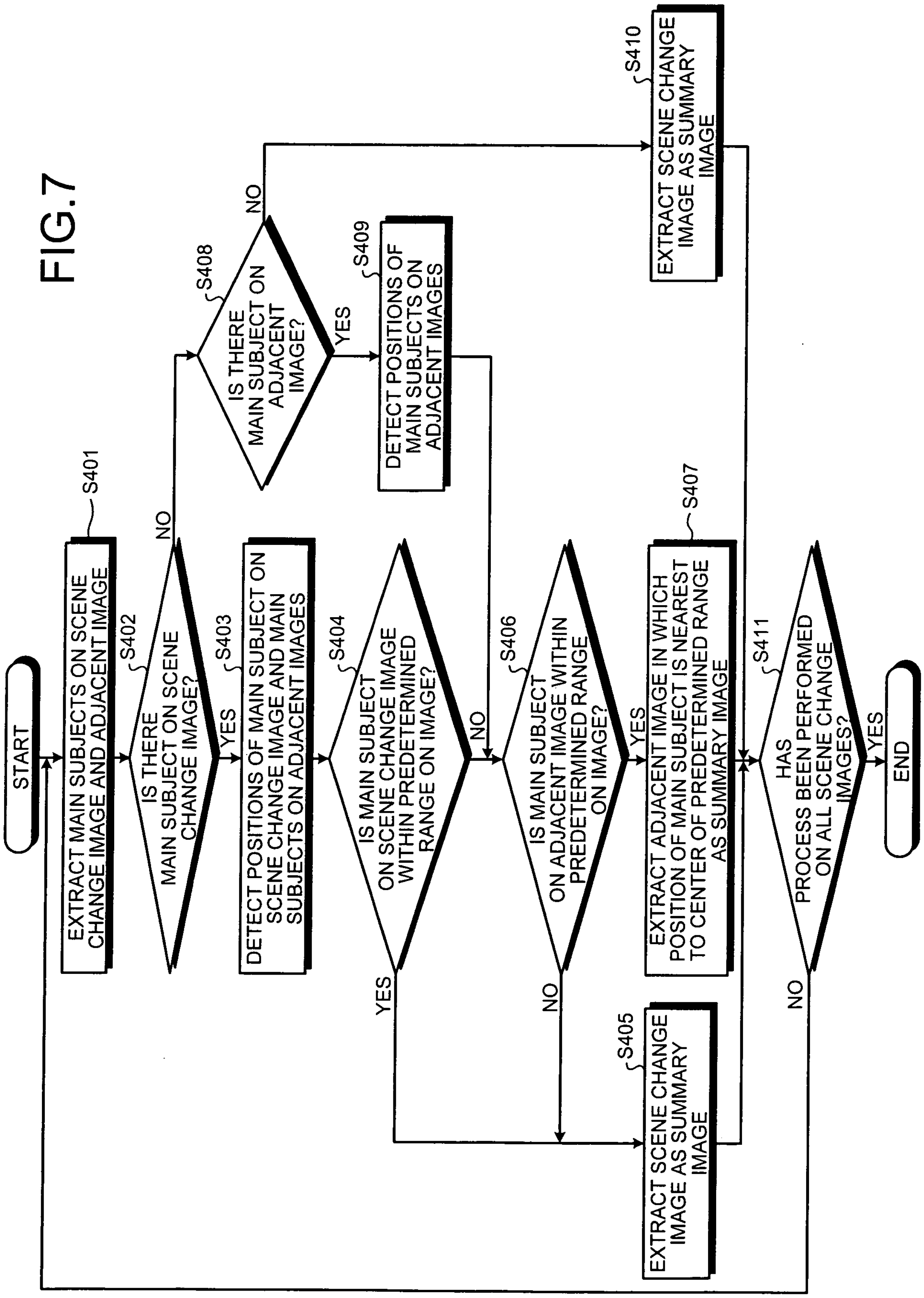
START
EXTRACT MAIN SUBJECTS ON SCENE CHANGE IMAGE AND ADJACENT IMAGE
S401
IS THERE MAIN SUBJECT ON SCENE CHANGE IMAGE?
S402
YES
NO
DETECT POSITIONS OF MAIN SUBJECT ON SCENE CHANGE IMAGE AND MAIN SUBJECTS ON ADJACENT IMAGES
S403
IS MAIN SUBJECT ON SCENE CHANGE IMAGE WITHIN PREDETERMINED RANGE ON IMAGE?
S404
YES
NO
EXTRACT SCENE CHANGE IMAGE AS SUMMARY IMAGE
S405
IS MAIN SUBJECT ON ADJACENT IMAGE WITHIN PREDETERMINED RANGE ON IMAGE?
S406
YES
NO
EXTRACT ADJACENT IMAGE IN WHICH POSITION OF MAIN SUBJECT IS NEAREST TO CENTER OF PREDETERMINED RANGE AS SUMMARY IMAGE
S407
IS THERE MAIN SUBJECT ON ADJACENT IMAGE?
S408
YES
NO
DETECT POSITIONS OF MAIN SUBJECTS ON ADJACENT IMAGES
S409
EXTRACT SCENE CHANGE IMAGE AS SUMMARY IMAGE
S410
HAS PROCESS BEEN PERFORMED ON ALL SCENE CHANGE IMAGES?
S411
YES
NO
END

FIG.8

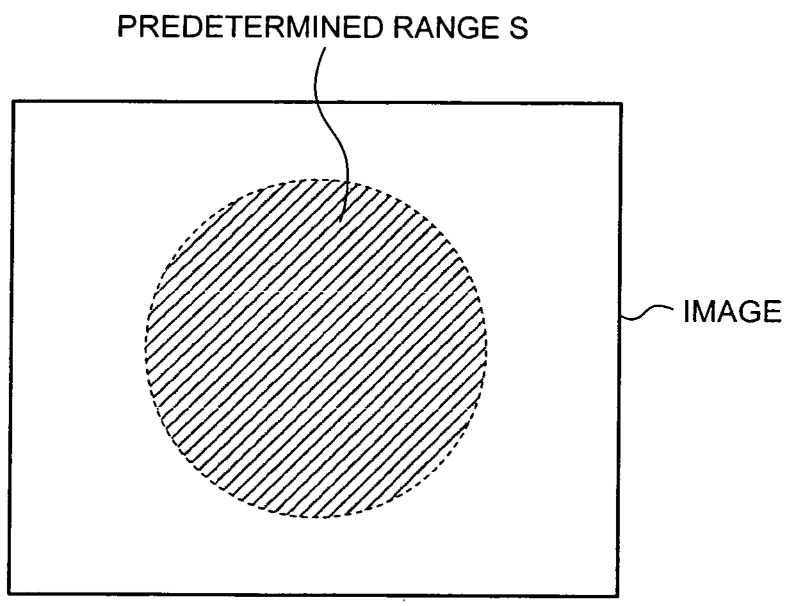

FIG.9

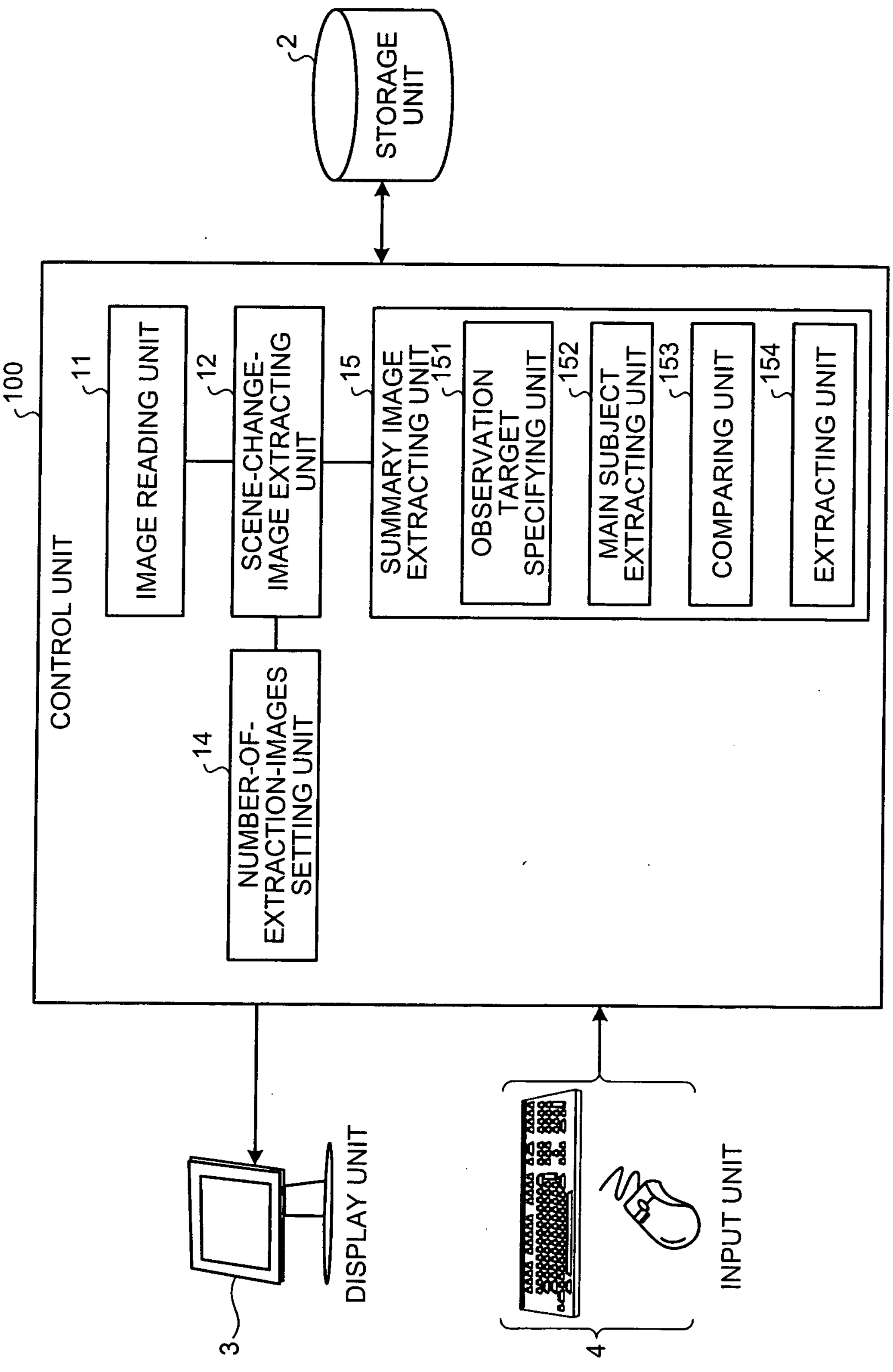
2
STORAGE UNIT
100
CONTROL UNIT
11
IMAGE READING UNIT
12
SCENE-CHANGE-IMAGE EXTRACTING UNIT
14
NUMBER-OF-EXTRACTION-IMAGES SETTING UNIT
15
SUMMARY IMAGE EXTRACTING UNIT
151
OBSERVATION TARGET SPECIFYING UNIT
152
MAIN SUBJECT EXTRACTING UNIT
153
COMPARING UNIT
154
EXTRACTING UNIT
3
DISPLAY UNIT
4
INPUT UNIT

FIG.10

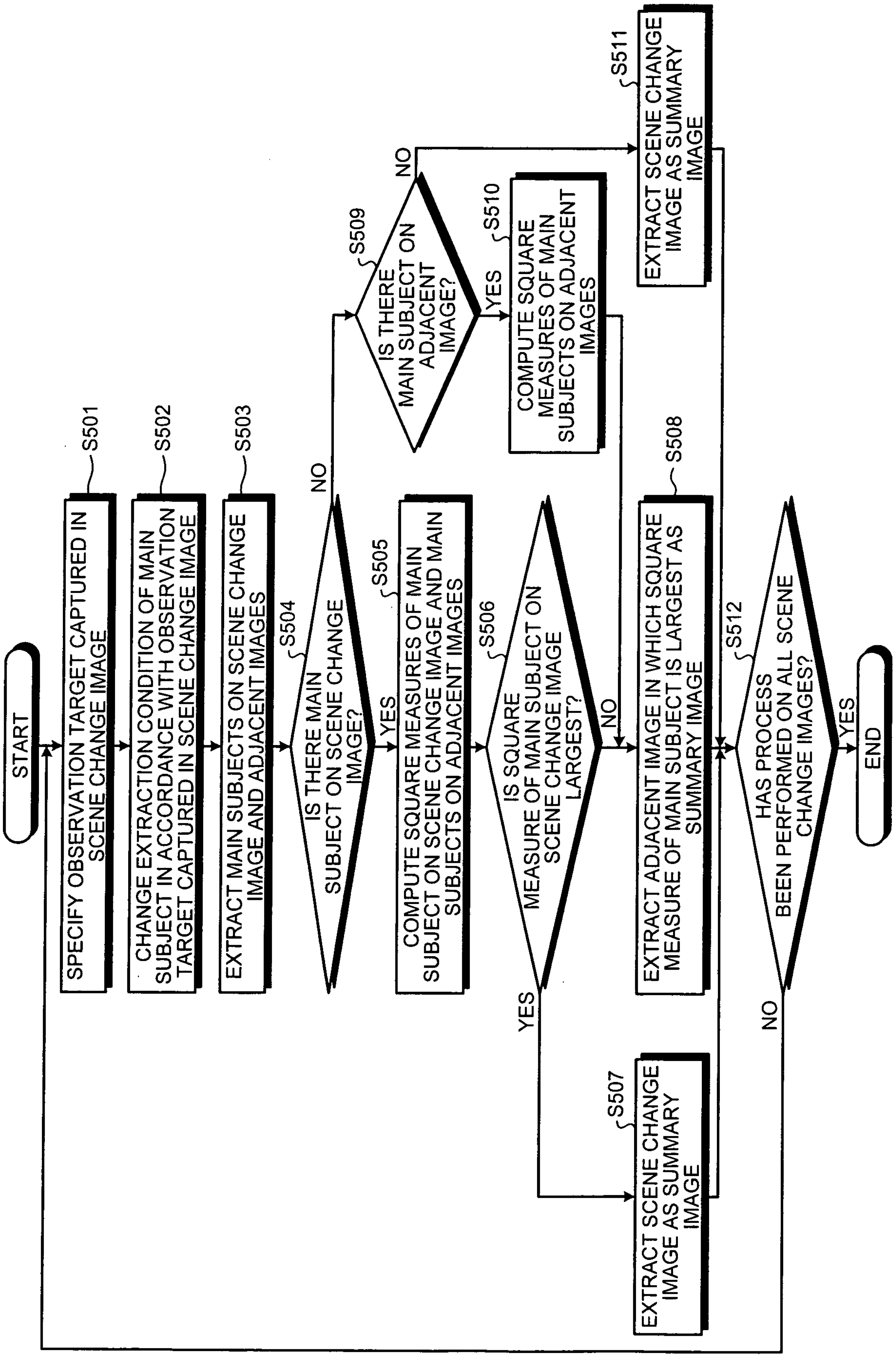
START
SPECIFY OBSERVATION TARGET CAPTURED IN SCENE CHANGE IMAGE
S501
CHANGE EXTRACTION CONDITION OF MAIN SUBJECT IN ACCORDANCE WITH OBSERVATION TARGET CAPTURED IN SCENE CHANGE IMAGE
S502
EXTRACT MAIN SUBJECTS ON SCENE CHANGE IMAGE AND ADJACENT IMAGES
S503
IS THERE MAIN SUBJECT ON SCENE CHANGE IMAGE?
S504
YES
NO
COMPUTE SQUARE MEASURES OF MAIN SUBJECT ON SCENE CHANGE IMAGE AND MAIN SUBJECTS ON ADJACENT IMAGES
S505
IS SQUARE MEASURE OF MAIN SUBJECT ON SCENE CHANGE IMAGE LARGEST?
S506
YES
NO
EXTRACT SCENE CHANGE IMAGE AS SUMMARY IMAGE
S507
EXTRACT ADJACENT IMAGE IN WHICH SQUARE MEASURE OF MAIN SUBJECT IS LARGEST AS SUMMARY IMAGE
S508
IS THERE MAIN SUBJECT ON ADJACENT IMAGE?
S509
YES
NO
COMPUTE SQUARE MEASURES OF MAIN SUBJECTS ON ADJACENT IMAGES
S510
EXTRACT SCENE CHANGE IMAGE AS SUMMARY IMAGE
S511
HAS PROCESS BEEN PERFORMED ON ALL SCENE CHANGE IMAGES?
S512
YES
NO
END

FIG.11

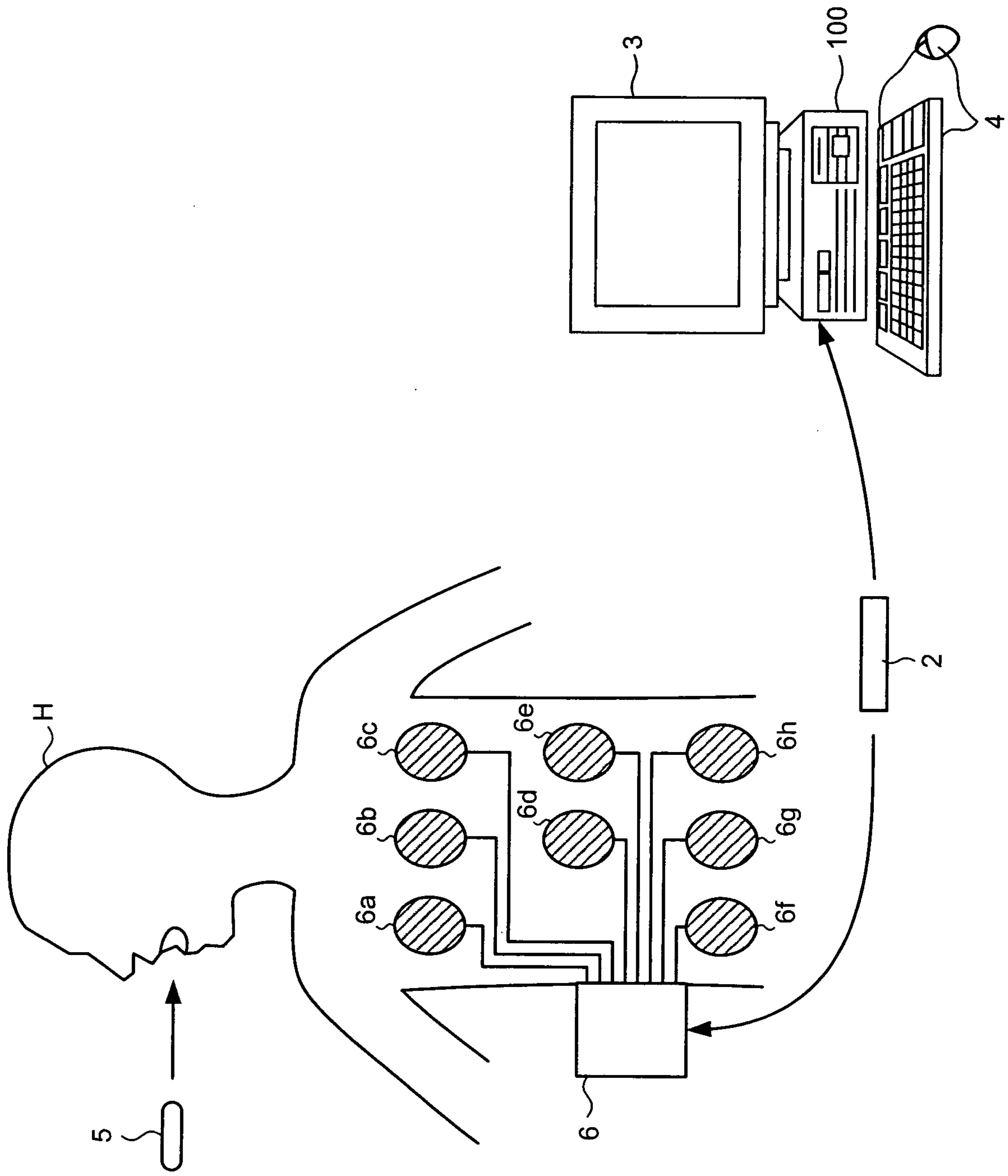

FIG.12

| | | MAIN SUBJECT PARAMETER (SIZE PARAMETER) | | |
|---|---|---|---|---|
| OBSERVATION TARGET | | STOMACH | SMALL INTESTINE | LARGE INTESTINE |
| IMAGING DISTANCE | 0.1 | 1.0 | 0.6 | 0.8 |
| | 0.2 | 0.8 | 0.5 | 0.7 |
| | . | . | . | . |
| | . | . | . | . |
| | 1.0 | 0.1 | 0.1 | 0.1 |

FIG.13

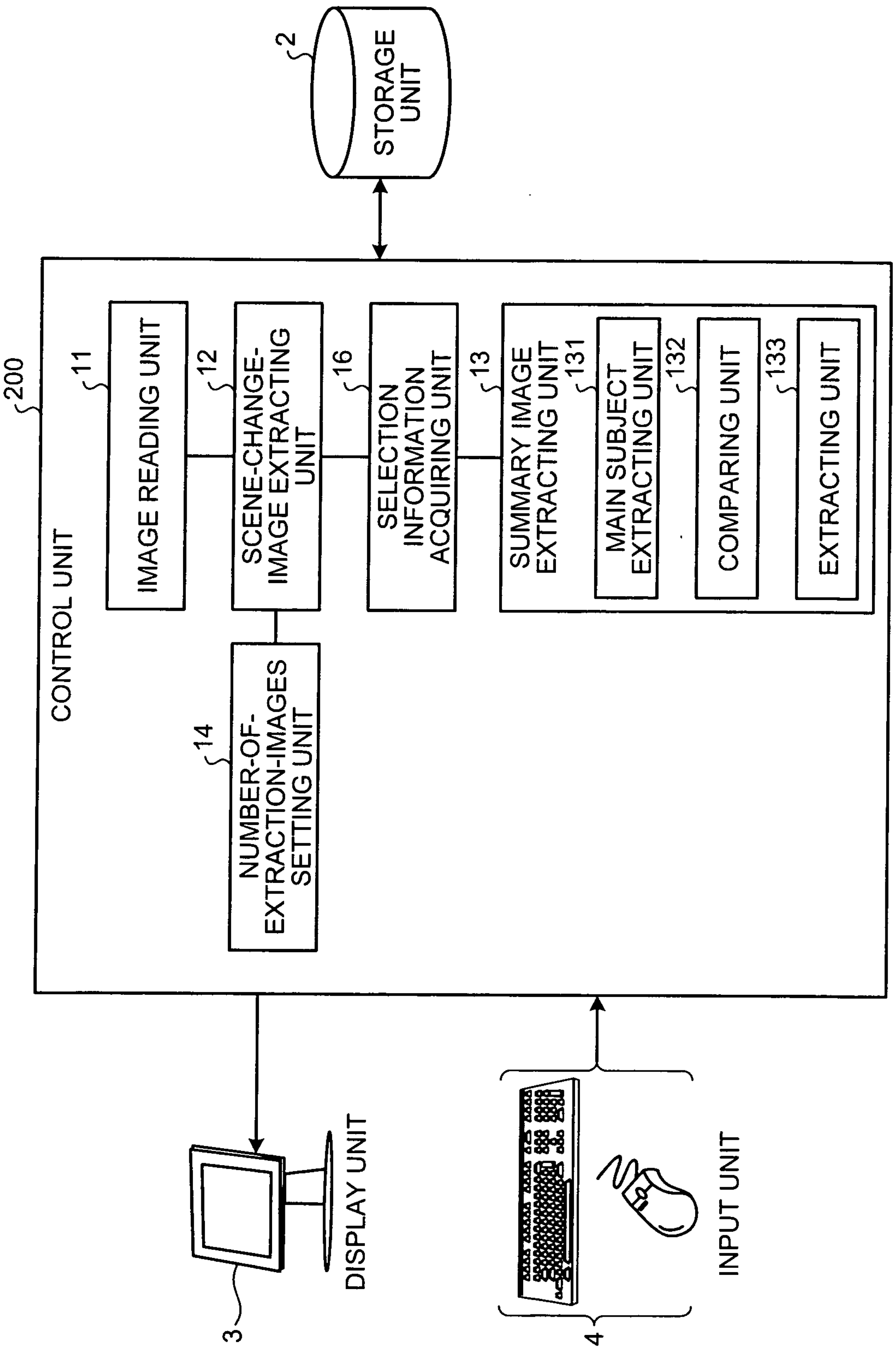

200
CONTROL UNIT
11
IMAGE READING UNIT
12
SCENE-CHANGE-IMAGE EXTRACTING UNIT
16
SELECTION INFORMATION ACQUIRING UNIT
13
SUMMARY IMAGE EXTRACTING UNIT
131
MAIN SUBJECT EXTRACTING UNIT
132
COMPARING UNIT
133
EXTRACTING UNIT
14
NUMBER-OF-EXTRACTION-IMAGES SETTING UNIT
2
STORAGE UNIT
3
DISPLAY UNIT
4
INPUT UNIT

FIG.14

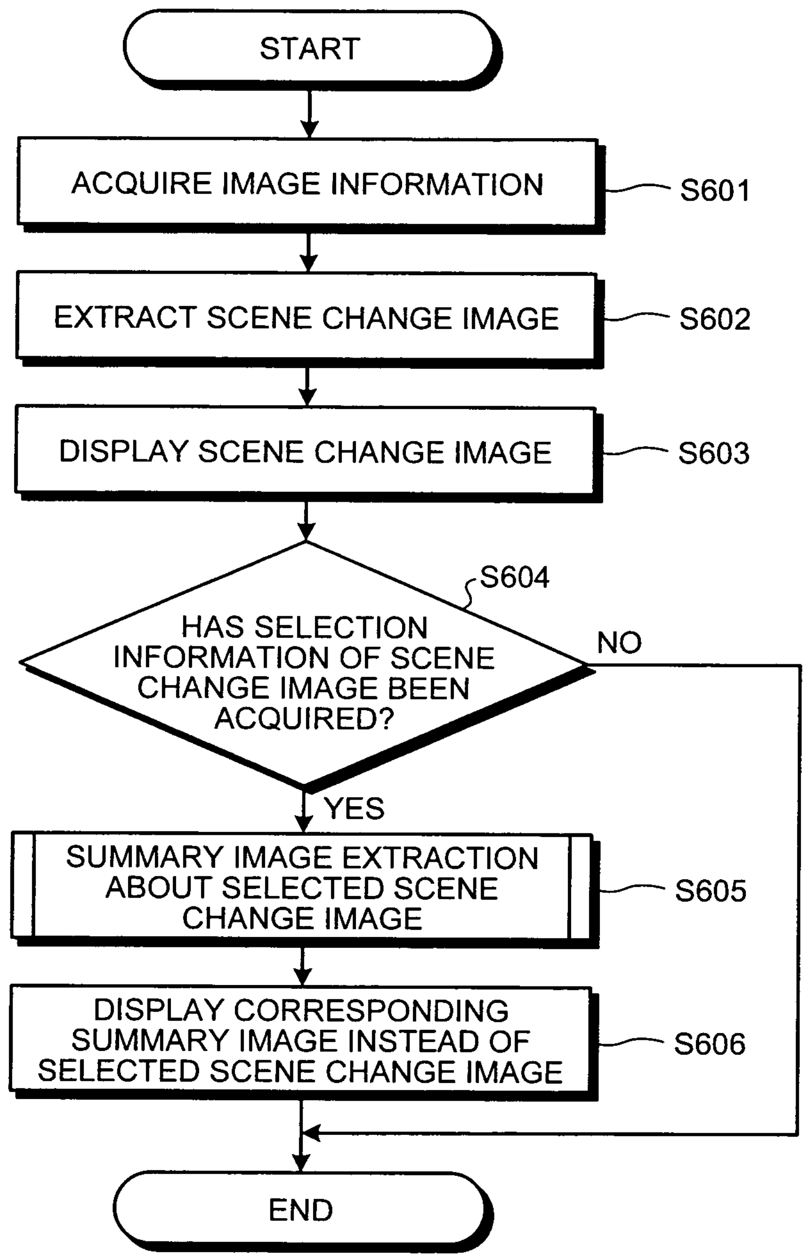
START
ACQUIRE IMAGE INFORMATION
S601
EXTRACT SCENE CHANGE IMAGE
S602
DISPLAY SCENE CHANGE IMAGE
S603
S604
HAS SELECTION INFORMATION OF SCENE CHANGE IMAGE BEEN ACQUIRED?
NO
YES
SUMMARY IMAGE EXTRACTION ABOUT SELECTED SCENE CHANGE IMAGE
S605
DISPLAY CORRESPONDING SUMMARY IMAGE INSTEAD OF SELECTED SCENE CHANGE IMAGE
S606
END

FIG.15

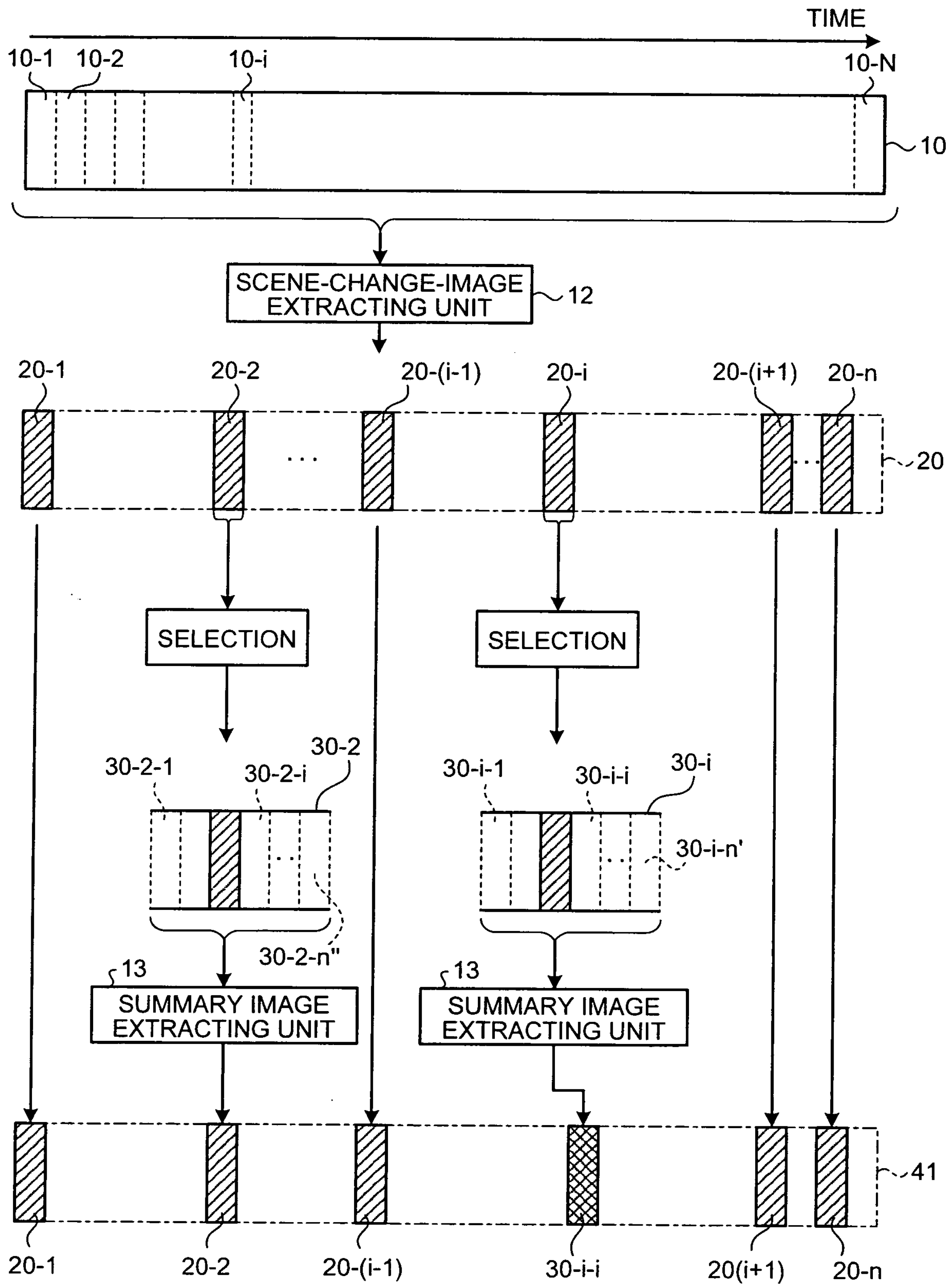
TIME
10-1
10-2
10-i
10-N
10
SCENE-CHANGE-IMAGE EXTRACTING UNIT
12
20-1
20-2
20-(i-1)
20-i
20-(i+1)
20-n
20
SELECTION
SELECTION
30-2-1
30-2-i
30-2
30-i-1
30-i-i
30-i
30-i-n'
30-2-n''
13
13
SUMMARY IMAGE EXTRACTING UNIT
SUMMARY IMAGE EXTRACTING UNIT
41
20-1
20-2
20-(i-1)
30-i-i
20(i+1)
20-n

FIG.16

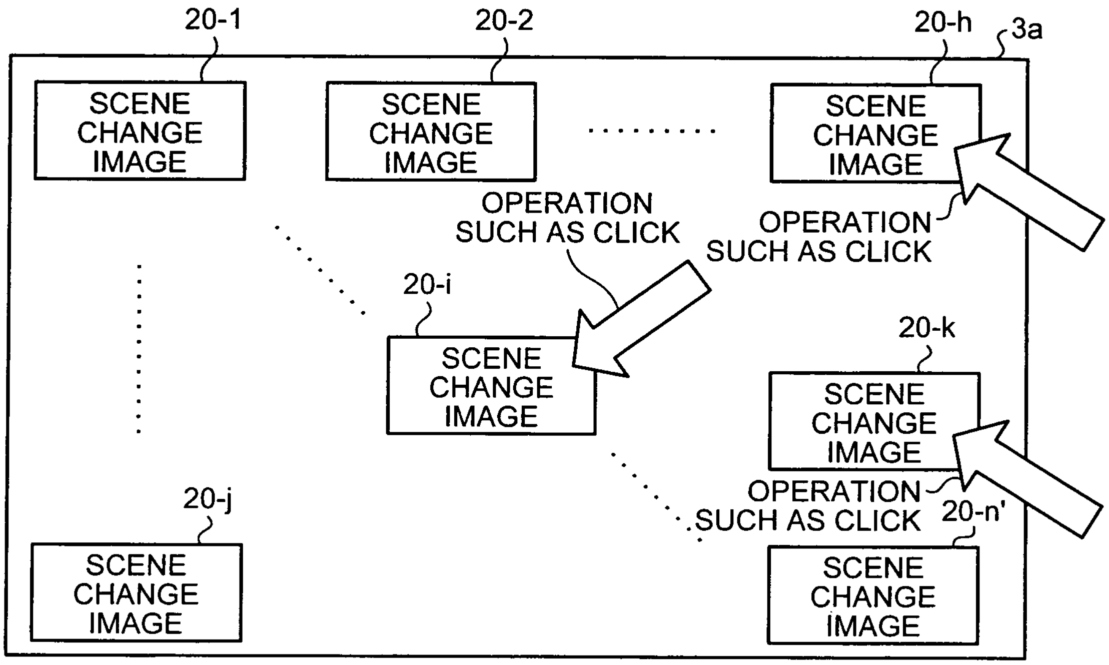
20-1
20-2
20-h
3a
SCENE CHANGE IMAGE
SCENE CHANGE IMAGE
SCENE CHANGE IMAGE
OPERATION SUCH AS CLICK
OPERATION SUCH AS CLICK
20-i
SCENE CHANGE IMAGE
20-k
SCENE CHANGE IMAGE
OPERATION SUCH AS CLICK
20-n'
20-j
SCENE CHANGE IMAGE
SCENE CHANGE IMAGE

FIG.17

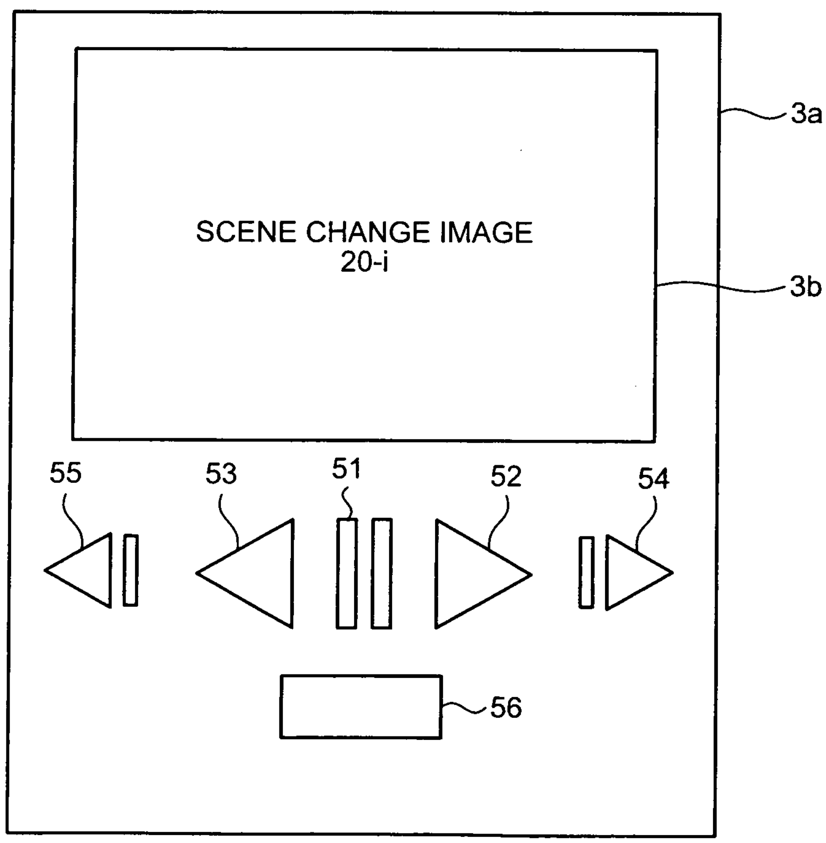
3a
SCENE CHANGE IMAGE 20-i
3b
55
53
51
52
54
56

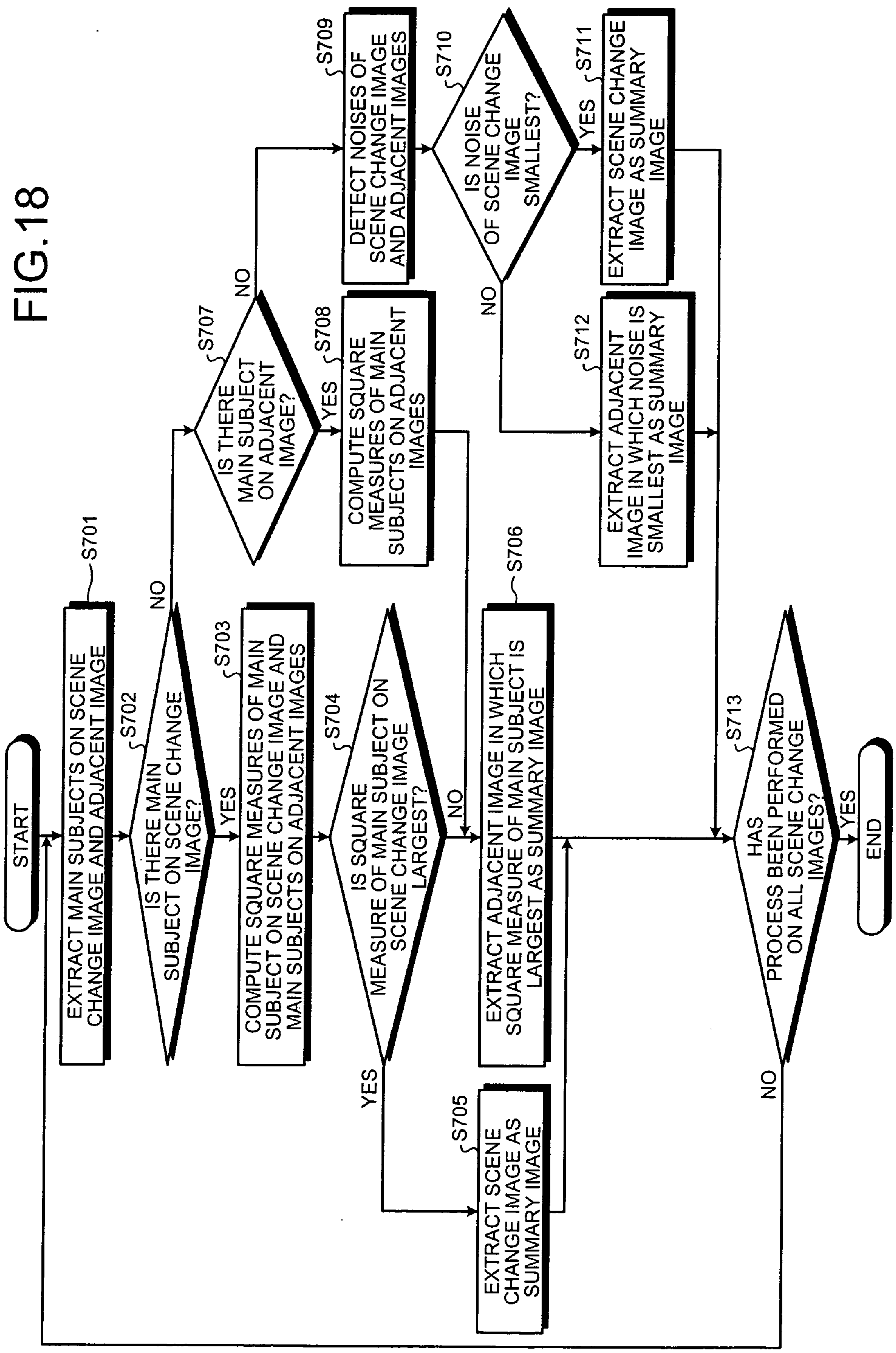
START
EXTRACT MAIN SUBJECTS ON SCENE CHANGE IMAGE AND ADJACENT IMAGE
S701
IS THERE MAIN SUBJECT ON SCENE CHANGE IMAGE?
S702
YES
NO
COMPUTE SQUARE MEASURES OF MAIN SUBJECT ON SCENE CHANGE IMAGE AND MAIN SUBJECTS ON ADJACENT IMAGES
S703
IS SQUARE MEASURE OF MAIN SUBJECT ON SCENE CHANGE IMAGE LARGEST?
S704
YES
NO
EXTRACT SCENE CHANGE IMAGE AS SUMMARY IMAGE
S705
EXTRACT ADJACENT IMAGE IN WHICH SQUARE MEASURE OF MAIN SUBJECT IS LARGEST AS SUMMARY IMAGE
S706
IS THERE MAIN SUBJECT ON ADJACENT IMAGE?
S707
YES
NO
COMPUTE SQUARE MEASURES OF MAIN SUBJECTS ON ADJACENT IMAGES
S708
DETECT NOISES OF SCENE CHANGE IMAGE AND ADJACENT IMAGES
S709
IS NOISE OF SCENE CHANGE IMAGE SMALLEST?
S710
YES
NO
EXTRACT SCENE CHANGE IMAGE AS SUMMARY IMAGE
S711
EXTRACT ADJACENT IMAGE IN WHICH NOISE IS SMALLEST AS SUMMARY IMAGE
S712
HAS PROCESS BEEN PERFORMED ON ALL SCENE CHANGE IMAGES?
S713
YES
NO
END

FIG.18

FIG.19

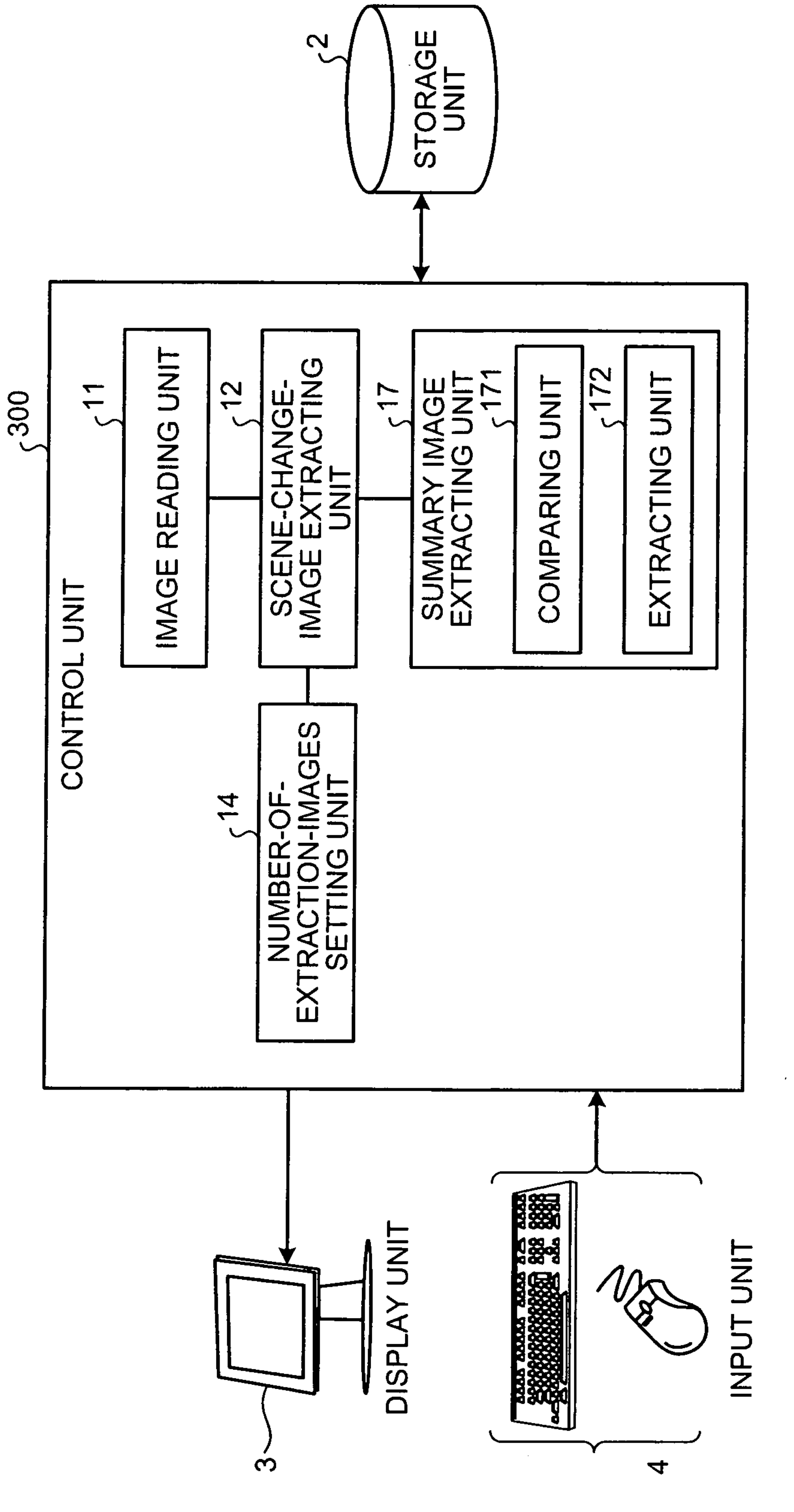
300
CONTROL UNIT
11
IMAGE READING UNIT
12
SCENE-CHANGE-IMAGE EXTRACTING UNIT
14
NUMBER-OF-EXTRACTION-IMAGES SETTING UNIT
17
SUMMARY IMAGE EXTRACTING UNIT
171
COMPARING UNIT
172
EXTRACTING UNIT
2
STORAGE UNIT
3
DISPLAY UNIT
4
INPUT UNIT

FIG.20

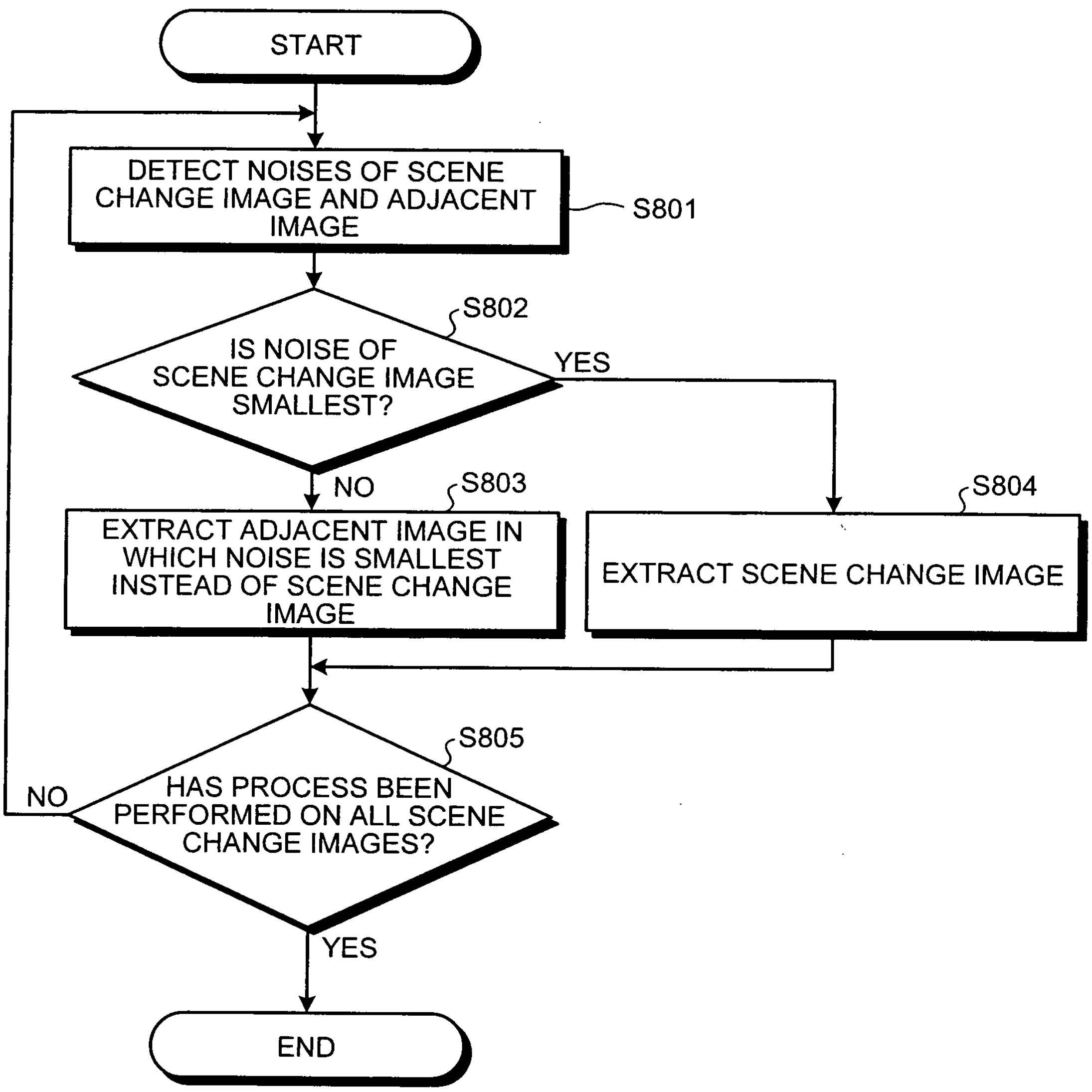
START
DETECT NOISES OF SCENE CHANGE IMAGE AND ADJACENT IMAGE
S801
IS NOISE OF SCENE CHANGE IMAGE SMALLEST?
S802
YES
NO
S803
EXTRACT ADJACENT IMAGE IN WHICH NOISE IS SMALLEST INSTEAD OF SCENE CHANGE IMAGE
S804
EXTRACT SCENE CHANGE IMAGE
S805
HAS PROCESS BEEN PERFORMED ON ALL SCENE CHANGE IMAGES?
NO
YES
END

## INTERNATIONAL SEARCH REPORT

International application No.
PCT/JP2008/059364

A. CLASSIFICATION OF SUBJECT MATTER
*H04N5/76*(2006.01)i, *A61B1/00*(2006.01)i, *A61B1/04*(2006.01)i, *A61B1/06*(2006.01)i

According to International Patent Classification (IPC) or to both national classification and IPC

B. FIELDS SEARCHED

Minimum documentation searched (classification system followed by classification symbols)
H04N5/76, A61B1/00, A61B1/04, A61B1/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2003-348528 A (Nippon Telegraph And Telephone Corp.), 05 December, 2003 (05.12.03), Full text; all drawings (Family: none) | 1-18 |
| A | JP 2005-45843 A (Hitachi, Ltd.), 17 February, 2005 (17.02.05), Full text; all drawings (Family: none) | 1-18 |
| A | JP 2006-155384 A (Nippon Telegraph And Telephone Corp.), 15 June, 2006 (15.06.06), Full text; all drawings (Family: none) | 1-18 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 June, 2008 (13.06.08) | 24 June, 2008 (24.06.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2001054055 A
- JP 2006217045 A
- JP 2006217046 A